# EUROPEAN PATENT SPECIFICATION

(11) **EP 2 292 731 B1**
(45) Date of publication and mention of the grant of the patent: **04.04.2018**
(21) Application number: 10172690.9
(22) Date of filing: 12.08.2010
(51) Int. Cl.: C12N 1/00, C12N 1/20, A23L 29/00, A23L 33/135, A23L 13/40

(54) **Method for preparing complex cultures**
Verfahren zur Herstellung von komplexen Kulturen
Procédé de production de cultures complexes

(30) Priority: 13.08.2009 EP 09010437
(43) Date of publication of application: 09.03.2011
(73) Proprietor: DuPont Nutrition Biosciences ApS, 1411 Copenhagen K (DK)
(72) Inventor: Budde-Niekiel, Andrea, 25712, Burg (DE); Friedrich, Udo, 25917, Leck (DE); Goelling, Detlef Uwe, D-25856, Hattstedt (DE); Gronau, Oliver, 22043, Hamburg (DE)
(74) Representative: DuPont EMEA

(56) References cited:
- EP-A1- 0 521 166
- WO-A1-02/089592
- WO-A1-03/067997
- RU-C1- 2 083 665
- US-A- 3 262 864
- US-A- 5 026 647

## Description

### Field of the Invention

The present invention relates to a method for preparing complex cultures of microorganisms.

### Description of the Prior Art

Complex cultures, for example Probat 505™ (a complex mesophilic dairy culture produced and sold by Danisco A/S), are cultures comprising different microorganisms (e.g. bacteria) that are co-cultivated. Complex cultures typically comprise a high and undefined diversity of strains and are also called traditional cultures since their origins go back to spontaneous fermentations of foodstuffs. Such complex cultures differ fundamentally from multiple strain cultures, the latter comprising a blend of a defined number of single strains. In such multiple strain cultures, each single strain is usually cultivated separately before blending, contrary to co-cultivation of an undefined number of strains in a complex culture.

Complex cultures are often used as starter cultures in the production of, for example foodstuffs, such as dairy products, e.g. cheese, meat based products or vegetable based foodstuffs. The complex culture may be a probiotic culture.

Boquien et al., Applied and Environmental Microbiology Oct 1988, p2527-2531, demonstrate the effects of fermentation conditions on growth of *Streptococcus cremoris* AM2 and *Leuconostoc lactis* CNRZ 1091 in pure and mixed cultures. Boquien *et al.* show that a starter culture is composed of strains with properties defined by their acidifying and flavour-developing properties, their sensitivity to bacteriophages, and their potential production of bacteriocins and bitter peptides. Boquien *et al* state that these characteristics are no longer sufficient to characterise the starter; and that it is necessary to study the behaviour of individual strains in mixed culture under different fermentation conditions to determine final populations, µₘₐₓ and acidification rates. Boquien *et al* do this by using a reactor in well-controlled culture conditions.

US5026647 discloses including a heavy metal salt in a medium comprising a mixed culture of *Propionibacterium* and other microorganisms to selectively promote the growth of metal salt-resistant *Propionibacterium* strains.

In complex cultures, each microorganism may provide a certain functionality to the final product. For example, the following organisms which are often present in complex cultures (such as Probat 505™) display the following main functionality/functionalities:
*Lactococcus lactis* subspecies *lactis:* acidification
*Lactococcus lactis* subspecies *cremoris*: acidification
*Lactococcus lactis* subspecies *lactis* biov. *diacetilactis*: aroma and gas formation
*Leuconostoc* ssp.: aroma and gas formation
The different organisms in a complex culture often have different growth requirements, but due to the fact that they are grown in co-culture in a complex culture the fermentation process typically compromises between the different needs of the different organisms.

One problem with this conventional co-cultivation method is that it may lead to suboptimal growth conditions and consequently lower cell count numbers and lower activity in the culture for one or more of the microorganisms. Additionally, minor variations in the fermentation process or in the raw materials used may affect the balance between the species and the strains and, consequently, the overall functionality of the complex culture.

In the production of complex cultures using conventional methods a consistent bacterial flora composition is difficult to achieve.

One aim of the present invention is to provide a flexible and/or efficient system for the production of complex cultures.

### Summary of the Invention

The present invention provides in a first aspect a method of preparing a blended complex culture of microorganisms according to claim 1 as appended. In a further aspect the present invention provides the use of two or more cultivated portions to manufacture a blended complex culture according to claim 17 as appended.

### Brief Description of the Drawings

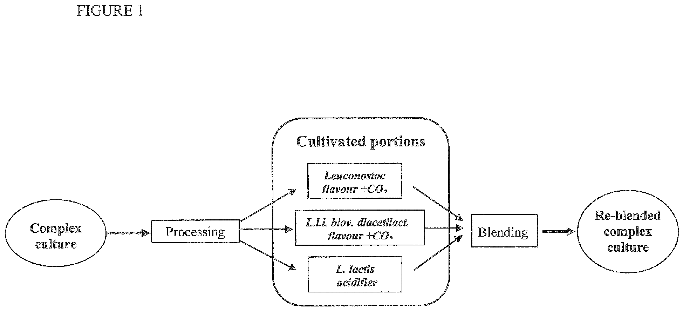

Figure 1 shows a schematic of one example of the method of the present invention, showing the cultivated portion (sometimes referred to herein as the "building block") approach to the production of complex cultures;
Figure 2 shows the cell counts and the ratio of the different cultivated portions *(Lactococcus lactis* (acidifier), *L. lactis* subsp. *lactis* biov. *diacetilactis* and *Leuconostoc)* in PROBAT 505™ at different fermentation temperatures [CFU/ml on calcium-citrate-agar];
Figures 3a and 3b shows the cell counts and % of total cell count of the different cultivated portions *(Lactococcus lactis* (acidifier), *L. lactis* subsp. *lactis* biov.
*diacetilactis* and *Leuconostoc)* in PROBAT 505™ at different pH set points [CFU/ml on calcium-citrate-agar];
Figures 4a and 4b show the influence of some tested compounds on the growth of *Lactococcus lactis (acidifier)* in PROBAT 505™ (a) [CFU/ml on calcium-citrate-agar], (b) % compared to the reference. Ref A and Ref B are two different batches of PROBAT 505™;
Figures 5a and 5b show the influence of some tested compounds on the growth of *Lactococcus lactis* subsp. *lactis* biov. *diacetilactis* in PROBAT 505™ (a) [CFU/ml on calcium-citrate-agar], (b) % compared to the reference. Ref A and Ref B are two different batches of PROBAT 505™;
Figures 6a and 6b show the influence of some tested compounds on the growth of *Leuconostoc ssp.* in PROBAT 505™ (a) [CFU/ml on calcium-citrate-agar], (b) % compared to the reference. Ref A and Ref B are two different batches of PROBAT 505™;
Figure 7 shows a consensus dendrogram of DNA-fingerprints of the isolates based on the primer EM6;
Figure 8 shows an electropherogram of a complex culture (a) and the cultivated portion *Lactococcus lactis* (acidifier) (b). Each peak refers to a DNA-fragment;
Figure 9 shows acidification curves of the re-blended culture compared to PROBAT 505™ (□); and
Figure 10 shows acidification curves of the blended phage alternative culture compared to PROBAT 505™ (□).

### Detailed Description of the Invention

The present invention relates to complex cultures - the term "complex culture" as used herein means a culture comprising more than one different microorganism (e.g. bacteria) that are co-cultivated. The complex culture used in the method according to the invention comprises at least 5, more preferably more than 5, for example at least 6, 7, 8, 9, or 10 different microorganisms which are co-cultivated.

It will be readily apparent to the skilled person that in general complex cultures comprise an undefined, and often high, diversity or number of strains.

These complex cultures are also called traditional cultures since their origins go back to spontaneous fermentations of foodstuffs. Such complex cultures differ fundamentally from multiple strain cultures, the latter comprising a blend of a defined number of single strains. In such multiple strain culture each single strain is usually cultivated separately before blending as opposed to co-cultivation of an undefined number of strains in a complex culture.

Suitably the term "taking at least two portions from an initial complex culture" as used herein may mean dividing an initial complex culture into at least two portions. This may mean that the initial complex culture is separated into equal portions (i.e. it is halved when there are two portions, or it is divided into thirds when there are three portions etc.). Alternatively, this may mean that the initial complex culture is separated into unequal portions. In some embodiments not all of the initial complex culture may be used. In other embodiments all of the initial complex culture will be used.

It will be further understood that the term "culturing each of the portions under conditions which enrich each portion" refers to culturing the at least two portions under different conditions in order to enrich each portion in one or more microorganism.

In a further embodiment, at least one of said portions may be subjected sequentially to at least two different sets of culture conditions so as to enrich the portion in one or more, preferably two or more, microorganisms. For example, the method of the present invention may comprise culturing one of said portions under a first set of conditions which enrich one or more microorganisms to form a first cultivated portion and subsequently culturing this first cultivated portion under a second set of conditions which further enrich one or more microorganisms to form a second cultivated portion and formulating a blended complex culture comprising said second cultivated portion. Preferably, the microorganisms enriched during culturing under the first and second sets of conditions are different.

Preferably the term "microorganism" as used herein includes bacteria, moulds and/or yeasts. In one preferred embodiment the terms "microorganism" and "microorganisms" as used herein mean bacterium and bacteria, respectively.

Suitably the term "microorganism" as used herein may be a microorganism genus, a species, a subspecies, a biovariety, or a strain.

In some embodiments the term "one or more microorganisms" as used herein may be one or more genera, one or more species, one or more subspecies, one or more biovarieties or one or more strains or any combination thereof.

Complex cultures typically contain a high number of different strains within each genus, species, subspecies or biovariety. In the present invention the strain complexity in the complex culture is maintained.

Therefore although we may refer to a specific genus, species, subspecies, biovariety or strain herein as a microorganism and the cultivated portion may be enriched for a specific genus, species, subspecies, biovariety or strain of a microorganism, it is envisaged that each genus, species, subspecies, or biovariety comprises a number of strains.

The portions in the present invention are cultured under conditions which enrich each portion in one or more microorganisms to obtain a cultivated portion. The term "enrich" or "enrichment" as used herein may mean increasing one microorganism (e.g. bacteria) in the culture compared with another microorganism (e.g. bacteria) in the culture or may mean increasing one microorganism (e.g. bacteria) in the portion compared with the amount (CFU/mL and/or %) of the same microorganism (e.g. bacteria) initially in the portion taken from the initial complex culture.

Suitably the term "enrich" or "enrichment" may mean increasing the actual number of microorganisms (e.g. bacteria).

In some embodiments one may wish to enrich for or increase the content of a specific mixture of microorganisms (e.g. bacteria), such as a specific mixture of different genera, species, subspecies, biovarieties or strains of microorganisms (e.g. bacteria) or combinations thereof.

In some embodiments preferably the cultivated portion (following culturing to enrich the portion in one or more microorganisms) may be enriched such that more than 80% (preferably more than 85%, more preferably more than 90%, more preferably more than 95%, suitably more than 98%, suitably 100%) of the microorganisms in the cultivated portion are comprised of the specific microorganism (e.g. bacteria) (or the specific mixture of microorganisms) being enriched.

Therefore, in one embodiment more than 80% (preferably more than 85%, more preferably more than 90%, more preferably more than 95%, suitably more than 98%, suitably 100%) of the microorganisms (e.g. bacteria) in the cultivated portion is comprised of a specific genus, species, subspecies, biovariety or strain of microorganism (e.g. bacteria) (or a specific mixture of genera, species subspecies, biovarieties or strains of microorganisms [e.g. bacteria]).

Each cultivated portion remains a complex culture, i.e. with a high strain diversity. Suitably, the amount of microorganisms in the cultivated portion is determined at the end of the culturing step, i.e. immediately after culturing each of the portions under conditions, which enrich each portion in one or more microorganisms to obtain a cultivated portion. Suitably the amount of the microorganism may be determined after culturing the portion between about 2 to about 76 hours total fermentation time.

The blended complex culture is formulated such that it comprises at least one of the cultivated portions (building blocks). A high strain diversity in the blended complex culture is maintained.

In some embodiments, the blended complex culture may have a different composition (such as a different number of microorganisms; or different mixture of microorganisms; or a different relative ratio of different microorganisms) compared with the initial complex culture. In other embodiments, the blended complex culture may be a re-blended complex culture, i.e. one where the blended complex culture has the same composition as the initial complex culture. One advantage of the latter embodiment is that as there is no cultivation compromise, i.e. in other words the individual microorganisms are grown under optimal conditions, one can then re-blend in the same proportions (e.g. in respect of microorganisms and/or in respect of the number of microorganisms) as in the initial complex culture thus forming a blended complex culture, which will have (or will maintain) the properties of the initial complex culture.

When we refer to the same composition as the initial complex culture this may be in terms of the types of genera, species, subspecies, biovarieties, or strains and/or the number of microorganisms (i.e. in each genus, species, subspecies, biovariety or strain of microorganism).

In some embodiments, the initial complex culture may comprise a number of microorganisms and it may be desirable to enrich all of these microorganisms. In another embodiment it may be desirable to enrich only a few of the microorganisms in the initial complex culture (for example due to market constraints or types of applications of the complex cultures). By way of example only, the initial complex culture may comprise 2 different genera of microorganism and at least 3 species and 3 strains of microorganism, but it may be only desirable to enrich 1 of the genera and 1 or 2 of the strains or species.

In some embodiments the method according to the present invention may further comprise the optional steps of examining one or more of i) the initial complex culture, ii) a portion or part thereof, iii) the cultivated portion and/or iv) the blended complex culture to determine the composition of microorganism(s) therein. This examination may take place before step a) and/or after step a) and/or after step b) and/or after step c).

Preferably the initial complex culture and/or the blended complex culture is/are a complex culture for use in a foodstuff or feed or as a starter culture in a foodstuff or feed. By way of example only, the initial complex culture and/or the blended complex culture may be a complex culture for use in dairy products, such as cheese or milk, or a complex culture for use in a meat based food product, or a complex culture for use in a vegetable food product or a complex culture for use in or as a probiotic composition, such as a probiotic complex culture for dairy products for instance, or a complex culture for use in biopreservation.

In one embodiment the initial complex culture and/or the blended complex culture may be a thermophilic or mesophilic complex culture.

In one embodiment the initial complex culture and/or the blended complex culture may be a thermophilic complex dairy culture, a thermophilic complex culture for meat based food products, a thermophilic complex culture for vegetable-based food products, a thermophilic complex probiotic culture or a thermophilic biopreservation complex culture.

In another embodiment the initial complex culture and/or the blended complex culture may be a mesophilic complex dairy culture, a mesophilic complex culture for meat based food products, a mesophilic complex culture for vegetable-based food products, a mesophilic complex probiotic culture or a mesophilic complex biopreservation culture.

Suitably step a) of the method according to the present invention may comprise taking at least 2 (suitably at least 3, at least 4, at least 5 or at least 6) portions of the initial complex culture. For instance the method according to the present invention may comprise dividing the initial complex culture into at least 2 (suitably at least 3, suitably at least 4, suitably at least 5, or suitably at least 6) portions. In a preferred embodiment the process may comprise taking at least 2, preferably 3, portions of the initial complex culture, e.g. dividing the initial complex culture into at least 2, preferably 3, portions. Preferably the method according to the present invention comprises the step of determining the conditions for culturing the portions by testing each portion or a part thereof and determining at least one critical parameter for each portion which enables enrichment of a microorganism or enables enrichment of a specific mixture of microorganism(s), and culturing each portion under (a) condition(s) which includes said at least one critical parameter.

In one preferred embodiment, each portion is cultured under conditions which include at least one critical parameter.

Suitably, each portion or a part thereof may be tested to determine at least two critical parameters (preferably at least 3 parameters, more preferably at least 4 parameters) which enable enrichment of a microorganism or enable enrichment of a specific mixture of microorganism(s). Suitably each portion may be cultured under conditions which include said at least two critical parameters (preferably at least 3 critical parameters, more preferably at least 4 critical parameters).

The method may suitably further comprise testing each portion or a part thereof to determine the effect on the microorganism(s) in the portion of one or more of the following parameters: pH; temperature; agitation speed; phosphate salts; aeration; citrate; acetate; glutathione; cysteine; addition of protectants, e.g. used during freezing; cooling time; freezing time; pelleting size; parameters for preserving the portion. Suitably the method further comprises analysing one or more of i) the initial complex culture, ii) a portion or part thereof, iii) the cultivated portion and/or iv) the blended complex culture to determine one or more of the group consisting of: microorganism diversity (e.g. genus, species, subspecies, biovariety or strain diversity); genetic relationships between the microorganisms in the portion (such as DNA-fingerprinting of the microorganism(s) in the portion); bacteriophage sensitivity; bacteriophage resistance of the microorganism(s); the presence of functional genes which may result in preferred or detrimental functional effect; the functional properties of the portion (such as bacteriocin producing properties for example).

Preferably the method comprises analysing ii) a portion of the initial complex culture or a part thereof.

Conventional techniques suitable for carrying out the analyses above will be known to the person skilled in the art and include the use of microbial and molecular biological methods such as DNA fingerprinting and/or DNA microarray techniques for example.

The method may additionally or alternatively comprise testing one or more of i) the initial complex culture, ii) a portion or part thereof, iii) the cultivated portion and/or iv) the blended complex culture to determine one or more of the following properties (or functionalities) selected from the group consisting of: rheological properties, aroma, taste, texture, viscosity, preserving properties of the portion, probiotic properties, acidification, gas formation, lactose reduction, bacteriophage sensitivity; bacteriophage resistance, health benefits (for example production of certain vitamins), production of substances that promote the growth of the culture and interaction between the microorganisms, production of substances that increase the survival of microorganisms after freezing and/or freeze drying, production of biopreservative substances, capacity to remove undesirable compounds by enzymatic activity (e.g. production or use of bitter peptides causing an off-flavour).

Preferably, the method comprises analysing ii) a portion of the initial complex culture or a part thereof.

In one embodiment the cultivated portion and/or the blended complex culture may be preserved. For example the cultivated portion or the blended complex culture may have one or more preservatives (e.g. chemical preservatives) added thereto; and/or the cultivated portion or the blended complex culture may be frozen (for example under a pelletized format); and/or the cultivated portion or the blended complex culture may be in liquid form (e.g. preserved in liquid form) and/or dried (such as freeze-dried (i.e. lyophilised) or spray-dried, or dried using any other preservation technique) and/or encapsulated (e.g. by micro-encapsulation or nano-encapsulation).

The cultivated portion may be formulated into a blended complex culture, i.e. step c), in a liquid, frozen, encapsulated or dried state or following thawing or rehydrating of the cultivated portion.

Suitably the method of the present invention may additionally comprise the step of freezing (and/or preparing a frozen block or pellet) and/or drying (such as freeze-drying (i.e. lyophilising) or spray-drying, or drying using any other preservation technique) and/or encapsulating at least one cultivated portion following step b), i.e. following culturing each portion under conditions which enrich each portion in one or more microorganisms to obtain cultivated portions.

In one embodiment the method according to the present invention may comprise the additional optional step:
b) i) freezing or drying (e.g. freeze-drying (i.e. lyophilising) or spray drying) or encapsulating the cultivated portion, following step b). In which case, the cultivated portion may be formulated into a blended complex culture, i.e. step c), in a frozen, encapsulated or dried state or following thawing or rehydrating of the cultivated portion.

Suitably the method of the present invention may comprise the step of freezing, drying (e.g. freeze-drying (i.e. lyophilising) or spray drying) and/or encapsulating the blended complex culture following step c), i.e. formulating a blended complex culture comprising at least one of said cultivated portions.

In one embodiment the method according to the present invention may comprise the additional optional step:
d) freezing, drying (e.g. freeze-drying (i.e. lyophilising) or spray drying) and/or encapsulating the blended complex culture.

In some embodiments the blended complex culture may be in the form of a powder (preferably a lyophilised powder), or in the form of a pellet, or in the form of a tablet, or in the form of a capsule or in the form of a liquid.

Suitably the cultivated portion(s) may be admixed or formulated into the blended complex culture as active (e.g. liquid) cultures, as frozen cultures or as lyophilised cultures. The cultivated portion(s) may be in the form of a powder, a tablet, a liquid, a capsule or a pellet. In one embodiment preferably the cultivated portion(s) are in the form of a powder, a tablet, a liquid, a capsule or a pellet prior to formulating said cultivated portion into the blended complex culture.

It will be understood that the complex cultures for use in the present invention are differentiated from multiple strain cultures or mixed strain cultures which comprise a predetermined number and combination of microorganisms which may be co-cultivated or cultivated individually and subsequently combined.

The term co-cultivated as used herein refers to the culture of at least two microorganisms together in a single growth medium.

The term "initial complex culture" as used herein refers to a complex culture which has not been further cultivated, enriched or blended by the addition of one or more microorganisms to the culture.

The term "cultivated portion" as used herein refers to a complex culture which has been cultivated under conditions which lead to the culture being enriched in one or more microorganisms.

It will be understood that as used herein the term portion does not refer to a single microorganism or strain which has been isolated from the complex culture by, for example, plating the complex culture and isolating single colonies or by any other suitable method.

The term "blended complex culture" as used herein refers to a culture comprising at least one cultivated portion of a complex culture and at least one further microorganism or culture thereof. This "at least one further microorganism or culture thereof" is a further cultivated portion of the complex culture or a different complex culture or cultivated portion thereof. The term "formulating a blended complex culture" refers to the step of mixing (admixing or blending) at least one of the cultivated portions with the at least one further microorganism or culture thereof.

It will be further understood that in one embodiment, the step of formulating the blended culture may occur subsequently to the step of culturing the portion(s) of the complex culture to form the cultivated portion(s).

In an alternative embodiment, the step of formulating the blended culture may be undertaken concomitantly with, or overlap with the culturing and enrichment step. It will be apparent to the skilled person that this could occur, for example, in a continuous rather than batch process.

In one embodiment the cultivated portion(s) may be formulated into a blended complex culture immediately following the production of the cultivated portion, i.e. immediately following the culturing step b). In some embodiments there may be a delay between production of the cultivated portion and formulating the cultivated portion(s) into the blended complex culture. In some embodiments the cultivated portions are concentrated (e.g. by filtration or centrifugation) prior to formulating the cultivated portion(s) into the blended complex culture. In some embodiments the cultivated portions may be frozen or dried (e.g. freeze-dried (i.e. lyophilised) or spray dried) or encapsulated prior to formulating the cultivated portion(s) into the blended complex culture.

In one embodiment the cultivated portion(s) may be formulated into a blended complex culture as and when the blended complex culture is placed into packaging. Thus in one embodiment the formulating of the blended complex culture may occur at the same time as packaging the blended complex culture. For example, the blending may be done as the cultivated portion(s) making up the blended complex culture (optionally with a microorganism or mixture thereof which has not been taken from the initial complex culture) are being placed into packaging, i.e. as the blended complex culture is placed into pouches or packaging for selling.

In one embodiment the present invention yet further provides a kit comprising at least two packages, each package comprising at least one cultivated portion.

In one embodiment of the present invention the formulation or blending step may take place prior to or during packaging of the blended complex culture. In one embodiment the blended complex culture may be formulated by placing an appropriate amount of each cultivated portion (optionally together with one or more cultures of at least one microorganism which has not been taken from the initial complex culture) in packaging to form a blended complex culture in the packaging.

In some embodiments preferably the blended complex culture is formulated prior to packaging the same.

The blended complex culture may be cultured under conditions which are the same as or similar to those used to culture the initial complex culture.

In some embodiments the blended complex culture is concentrated (e.g. by filtration or centrifugation) after the cultivated portions are admixed.

The cultivated portion and/or blended complex culture according to the present invention may be in the form of a frozen, dried (e.g. freeze-dried (i.e. lyophilised) or spray dried), liquid or encapsulated culture.

The blended complex culture may be sold in the form of a frozen, dried (e.g. freeze-dried (i.e. lyophilised) or spray dried), liquid or encapsulated culture. In one embodiment the blended complex culture may be sold in the form of a pellet, e.g. a freeze-dried (i.e. lyophilised) pellet, a powder, a tablet, a liquid or a capsule.

The blended complex culture may be used to inoculate a bulk starter culture which may then be used for inoculation of production tanks. Alternatively (and preferably), the blended complex culture may be used as a starter culture directly. In this way the advantages of the present invention may be fully maintained.

Preferably once the blended complex culture has been reactivated if necessary (i.e. thawed or rehydrated) it is used immediately or with minimal further culturing. In this way the advantages obtained using the present invention will be fully maintained.

In one preferred embodiment the blended complex culture of the present invention is a blended complex starter culture.

In one embodiment suitably each of the portions may be cultured under conditions which enrich each portion in two or more microorganisms (e.g. bacteria), suitably three or more (e.g. four or more, five or more, or six or more) microorganisms (e.g. bacteria).

In another embodiment suitably each of the portions is cultured under conditions which enrich a specific mixture of microorganisms (e.g. bacteria).

In one embodiment the one or more microorganisms or mixture of microorganisms in each cultivated portion are different compared with the other cultivated portion(s). The term "different" as used herein means that the actual genus, species, subspecies, biovariety or strain of microorganism (or mixture thereof) is different in each cultivated portion and/or that each cultivated portion has a different proportion or number of specific genera, species, subspecies, biovarieties or strains of microorganisms compared with other cultivated portions. By way of example, the aim is to achieve a ratio of more than 90% (preferably more than 91%, preferably more than 92%, preferably more than 93%, preferably more than 94%, even more preferably more than 95%) of the targeted genus, species, subspecies, biovariety or group of strains in one cultivated portion whereas the ratio of the same genus, species, subspecies, biovariety or group of strains in the other portion is less than 10% (preferably less than 9%, preferably less than 8%, preferably less than 7%, preferably less than 6%, even more preferably less than 5%), both portions being cultivated from the same original complex culture. Preferably, at least two portions are cultured under a different culture condition(s). Preferably, each portion is cultured under a different culture condition(s) compared with the other portion(s). Each portion is cultured under a condition(s) which promotes the growth of (and thus enriches the portion in) a microorganism (e.g. bacteria) or a mixture of microorganisms (e.g. bacteria) which has/have a specific functionality. Each cultivated portion imparts a different functionality on the blended complex culture. The term "cultivated portion" as used herein is used synonymously with the terms "building block". Each portion is cultured under a different culture condition(s) compared with the other portion(s), such that each cultivated portion imparts a different functionality on the blended complex culture. For instance, one portion may be cultivated to impart a texturising property on the blended complex culture, whereas another portion may be cultivated to impart a flavour or aroma property on the blended complex culture.

Suitably, the functionality may be selected from one or more of the group consisting of: probiotic properties, acidification, aroma enhancement. gas formation, texturisation, lactose reduction, nitrate reduction, rheological properties, taste, texture, viscosity, bacteriophage sensitivity, bacteriophage resistance, health benefits (for example production of certain vitamins), producer of substances that promote the growth of the culture and interaction between the species, producer of substances that increase the survival of strains after freezing and/or freeze drying, producer of biopreservative substances, capacity to remove undesirable compounds by enzymatic activity (e.g. production or use of bitter peptides causing an off-flavour).

In one embodiment of the present method the enrichment of one or more microorganisms (e.g. bacteria) in each portion is such that more than 80% (preferably more than 85%, more preferably more than 90%, more preferably more than 95%) of the microorganisms (e.g. bacteria) in the cultivated portion is comprised of the enriched microorganisms (e.g. bacteria). Alternatively, the at least one cultivated portion may be admixed with a further complex culture or portion thereof or cultivated portion thereof (e.g. a cultivated portion obtained from culturing a portion of a second complex culture which may be different from the initial complex culture).

In some embodiments, the blended complex culture may be a mixture of at least 2 or at least 3 or at least 4 of the cultivated portions, and optionally at least one microorganism (preferably a bacterium) which has not been taken from the initial complex culture and/or a further complex culture or portion thereof or cultivated portion thereof (which further complex culture may be different from the initial complex culture).

The culture of a single microorganism (preferably a bacterium) or a mixture of microorganisms (preferably bacteria) which has not been taken from the initial complex culture may for example comprise a bacteriophage resistant bacterium. Preferably when formulating the blended complex culture comprising at least one of said cultivated portions said method comprises admixing at least two (suitably at least 3, suitably at least 4, suitably at least 5) of said cultivated portions.

Suitably, formulating a blended complex culture comprising at least one of said cultivated portions preferably comprises admixing all of the cultivated portions obtained from culturing all of the portions taken from the initial complex culture. Thus the blended complex culture may have the same composition as the initial complex culture. Alternatively, the blended complex culture may be formulated by admixing all of the cultivated portions obtained from culturing all of the portions taken from the initial complex culture together with optionally a culture of at least one microorganism (preferably a bacterium) which has not been taken from the initial complex culture. For instance, all of the cultivated portions may be admixed together, as well as with a culture of a single microorganism (e.g. a single bacterium). Suitably, the single microorganism (e.g. bacterium) may not be one obtained from the initial complex culture.

Alternatively, or in addition, all of the cultivated portions may be admixed together, as well as with a further complex culture or portion thereof or cultivated portion thereof (e.g. obtained from a second complex culture which may be different from the initial complex culture).

In one embodiment each cultivated portion is admixed such that the microorganism(s) (e.g. bacteria) contained in the blended complex culture are in a specific ratio (this ratio can be the same as or different from the ratio of microorganisms found in the initial complex culture). The ratio may depend on the functional properties imparted by each cultivated portion and the requisite properties of the final blended complex culture. The selected ratio may be anywhere from 0.1 to 99.9%, preferably 1 to 99%. By way of example only the microorganism(s) (e.g. bacteria) contained in the blended complex culture may be in a ratio of 50:50, suitably 20:80 or 10:90 for example where there are two microorganisms. The ratio of each microorganism can be determined by known conventional methods, for example by way of microbiological cultivation on differential agar plates or by molecular-biological methods.

In one embodiment the method may comprise adding either the cultivated portion(s) (of step b) or the blended complex culture (of step c) to a food ingredient to produce a food product. Suitably the food product may be one or more of a dairy product, a meat based food product, a vegetable food product, a probiotic composition or a biopreservation composition.

In one particular embodiment, the present invention provides a method of preparing a blended complex culture (preferably a blended dairy complex culture) comprising:
a) dividing an initial complex culture into at least two portions, suitably at least three portions;
b) culturing each portion under conditions which enrich each portion in a specific bacterium or in a specific mixture of bacteria; wherein the bacteria enriched in each portion provide a specific functionality in the blended complex culture; and wherein in the obtained cultivated portion more than 80% (preferably more than 85%, more preferably more than 90%, more preferably more than 95%) of the bacteria in the cultivated portion is comprised of the enriched bacteria or bacteria mix;
c) formulating a blended complex culture comprising at least one of said cultivated portions (preferably the formulating comprises admixing two or more of the cultivated portions.

### ADVANTAGES

One advantage of the present invention is that it provides a flexible system for the production and development of complex cultures. For example the present invention provides a building block approach (e.g. cultivated portion approach) to formulating blended complex cultures.

A further advantage of the present invention is that each microorganism (e.g. bacterium) or only selected microorganisms may be enriched in the final complex culture, such as at least a specific genus, species, subspecies, biovariety or strain thereof (e.g. groups of strains).

An advantage of the present invention is the development of new complex cultures by blending of cultivated portions, these may be different from initial complex cultures. The new approach allows the flexible handling and development of blended complex cultures. By way of example bacteriophage-alternatives can easily be created by exchanging one phage sensitive cultivated portion from one complex culture by the same but phage resistant cultivated portion of another complex culture.

The new approach allows the creation of complex cultures with new functionalities without losing the complex culture status.

For example an advantage of the present invention is that it is possible to develop bacteriophage backups. Bacteriophage backups are alternative cultures to known cultures with the same functionality but which are not sensitive to bacteriophages. For example the present invention allows for a bacteriophage-sensitive cultivated portion or a microorganism (e.g. bacterium) infected by bacteriophages to be readily removed from complex cultures. This offers more possibilities to create bacteriophage resistant complex cultures without losing the complex status in terms of strain diversity in a complex culture. Parts of a culture that are bacteriophage-resistant may remain in the culture and only the sensitive parts may be exchanged. Thus, in one embodiment the present invention provides a simple method to remove a bacteriophage-sensitive cultivated portion or a simple method for replacing bacteriophage infected bacteria in a complex culture with uninfected bacteria or a bacteriophage resistant bacterium. The uninfected replacement bacteria or bacteriophage resistant bacteria may or may not have the same functionality as the bacteriophage infected bacteria.

Another advantage of the present invention is that it allows the strain complexity in each cultivated portion to be kept. Indeed, in the present invention a complex culture is taken as a basis for the cultivated portions. Therefore, the strain complexity, i.e. the main key feature of a complex culture, can be maintained in each cultivated portion. There is still high strain diversity at each step of the present method (i.e. in the initial complex culture, in the cultivated portion and in the blended complex culture).

One further advantage of the present invention is that the method enables the simple development of new complex cultures. The present invention may be used to develop bespoke complex cultures. The present invention may also be used to create blended complex cultures according to market needs. Currently the ratio of the cultivated portions in a complex culture can only be adapted within certain limits, with the new approach any ratio of the cultivated portions that is required can be achieved by blending.

Yet another advantage of the present invention is that an improvement in the consistency and yields of the complex culture can be readily achieved. By applying specific culturing conditions to the different bacterial groups or genera, species, subspecies, biovarieties or strains in a complex culture by using a cultivated portion (i.e. building block) approach the growth of each group is optimised leading to higher cell counts and better activity. In particular, without wishing to be bound by theory the needs of the different groups in a complex culture are different and sometimes contradictory, for example some strains prefer 30°C whilst others prefer only 20°C. In conventional production of a complex culture it has previously been necessary to compromise, for example incubate at 25°C, but this temperature would be actually sub-optimal for both groups. By compromising on the growth conditions suboptimal growth of each group has often thus far been achieved.

The present invention also leads to the advantageous reduction in production costs. For example, by achieving higher cell counts and better activity the total amount of cultures used for the blending can be reduced. For example instead of 500 g, 450 g might be sufficient in the packaged product.

A further advantage which has been observed is an improved product consistency. When a complex culture is fermented/produced in the traditional/conventional way (one step fermentation of the whole culture) the ratio of the specific bacterial groups can only partly be influenced during the fermentation. When the fermentation is completed the ratio of the different groups is given and sometimes the demanded ratio is not achieved. With the new approach every wanted ratio of the cultivated portions can be achieved by blending the cultivated portions.

### FUNCTIONALITY

Suitably each portion is cultured under condition(s) which promotes the growth of (and thus enriches a portion in) a microorganism or a mixture of microorganisms which has/have or impart(s) a specific functionality.

In one embodiment preferably each cultivated portion imparts a specific functionality on the blended complex culture. Suitably each cultivated portion imparts a different functionality on the blended complex culture compared with the other cultivated portions. However, in some embodiments more than one cultivated portion may impart the same functionality on the blended complex culture.

For example, the cultivated portion may impart one or more of the following functionalities on the blended complex culture or the final foodstuff in which the blended complex culture is used: acidification, aroma formation, lactose reduction, nitrate reduction, gas formation, a probiotic effect, flavour, mildness, consistency, texture, body, mouthfeel, firmness, viscosity, gel fracture, structure, bacteriophage sensitivity, bacteriophage resistance, health benefits (for example production of certain vitamins), production of substances that promote the growth of the culture and interaction between the species, production of substances that increase the survival of strains after freezing and/or freeze drying, production of biopreservative substances and/or organoleptic properties, capacity to remove undesirable compounds by enzymatic activity (e.g. production or use of bitter peptides causing an off-flavour). At least two portions are cultured under a different culture condition(s). Preferably different fermentation or culturing parameters are selected for each portion in order to obtain separate functionality. Thus at least two and preferably each cultivated portion provides a separate functionality

The parameters used to obtain the separate functionality, may be any parameter which affects the growth or metabolism of a microorganism. For example the parameter(s) may be selected from one or more of the following: aeration, such as an oxygen content of between about 50% to about 80% for example; pH; temperature; agitation speed; cooling time; freezing time; pelleting size; preservation parameters; phosphate salts; citrate content/presence; acetate content/presence; glutathione content/presence; cysteine content/presence.

As one skilled in the art would understand firstly it is necessary to determine what culture parameters influence the growth of a microorganism in the portion(s) and to determine which culture parameter(s) enhance(s) the growth or viability or survivability of microorganisms which are capable of imparting specific functional qualities on the portion, and then to use the selected culture parameter(s) when culturing the microorganisms in the portion(s).

In this way, one can produce an "enhanced" complex culture, e.g. blended complex culture. In one embodiment the invention relates to a process of taking one (initial) complex culture and carrying out the method of the present invention to produce an enhanced (blended) complex culture.

### BUILDING BLOCKS

The cultivated portion(s) taught herein are also referred to as building blocks. Thus the present invention relates to the use of building blocks comprising portions of an initial complex culture to produce an enhanced, blended complex culture.

### MICROORGANISMS

The microorganism of the present invention may be a bacterium, a yeast, a fungi or a mould for example, or mixtures thereof.

Some examples of yeasts, fungi and moulds are: *Geotricum candidum, Penicillium roqueforti, Penicillium candidum, Penicillium nalgiovense, Penicillium chrysogenum, Debaryomyces hansenii, Verticillium lecanii, Trichothecium domesticum, Candida famata, Candida utilis, Candida kefyr, Rhodosporidium infirmomimiatum, Pichia membranifaciens, Kluyveromyces lactis, Hafnia alvei.*

In one preferred embodiment, the microorganism used herein is a bacterium. Suitably the bacterium may be a lactic acid bacterium, a *Micrococcus (Micrococcus varians, Micrococcus luteus),* a bacterium having anti-microbial activity (such as anti-*Listeria* or anti-mould activity) or a bacterium having a nitrate reductase activity such as certain bacteria of the *Staphylococcus* genus, or any mixture thereof.

Preferably the bacterium is a lactic acid bacterium. In this specification the term 'lactic acid bacterium' includes any bacterium capable of producing, as the major metabolic end product of carbohydrate fermentation, lactic acid or at least one of its derivatives (including, but not limited to, propionic acid): the term is therefore intended to include propionic acid bacteria (PAB), which produce propionic acid as a carbohydrate fermentation product.

Preferably the bacterium for use in the present invention is a lactic acid bacterium which is generally recognised as safe and, which is preferably GRAS approved.

A skilled person will readily be aware of specific species and or strains of bacteria from within the genera described herein which are used in the food and/or agricultural industries and which are generally considered suitable for human and/or animal consumption.

Preferably, the bacterium used in accordance with the present invention is one which is suitable for human and/or animal consumption.

In the present invention, the bacteria used may be of the same type (genus, species, subspecies, biovariety, strain) or may comprise a mixture of genera, species, subspecies, biovarieties or groups of strains.

Suitable lactic acid bacteria may be selected from the genera *Lactococcus, Lactobacillus, Leuconostoc, Bifidobacterium, Carnobacterium, Enterococcus, Propionibacterium, Pediococcus,* and *Streptococcus* and mixtures thereof. Typically, the lactic acid bacteria are selected from the species, subspecies or biovarieties *Leuconostoc spp., Lactococcus lactis* subsp. *cremoris, Lactococcus lactis* subsp. *lactis, Lactococcus lactis* subsp. *lactis biovariety diacetilactis, Lactobacillus acidophilus, Lactocbacillus alimentarius, Lactobacillus casei, Lactobacillus coryneformis, Lactobacillus gasseri, Lactobacillus kefiri, Lactobacillus brevis, Lactobacillus helveticus, Lactobacillus paracasei ssp, Lactobacillus rhamnosus, Lactobacillus salivarius, Lactobacillus curvatus, Lactobacillus delbrueckii* subsp. *bulgaricus, Lactobacillus delbrueckii* subsp. *lactis, Lactobacillus delbrueckii* subsp. *delbrueckii, Lactobacillus sakei, Lactobacillus reuteri, Lactobacillus fermentum, Lactobacillus farciminis, Lactobacillus plantarum, Lactobacillus paraplantarum, Lactobacillus crispatus, Lactobacillus gasseri, Lactobacillus johnsonii, Lactobacillus jensenii, Lactobacillus animalis, Lactobacillus pentosus, Lactobacillus franciscensis, Bifidobacterium animalis* subsp. *lactis, Bifidobacterium bifidium, Bifidobacterium longum, Bifidobacterium animalis subsp. animalis, Bifidobacterium breve, Bifidobacterium infantis, Bifidobacterium catenulatum, Bifidobacterium pseudocatenulatum, Bifidobacterium adolescentis, Bifidobacterium angulatum,* and combinations of any thereof.

Preferably, the bacteria used in the present invention are selected from the genera *Lactococcus* or *Leuconostoc* and mixtures thereof. More preferably, the bacteria used in the present invention are selected from the species, subspecies or biovariety *Lactococcus lactis* subspecies *lactis, Lactococcus lactis* subspecies *cremoris, Lactococcus lactis* subspecies *lactis* biov. *diacetilactis, Leuconostoc* ssp, and mixtures thereof.

In preferred embodiments, the microorganism used in the present invention may be a probiotic microorganism. The probiotic strains most commonly used, in particular in dairy products, are principally bacteria and yeasts of the following genera: *Lactobacillus, Streptococcus., Enterococcus, Bifidobacterium.* and *Saccharomyces* In preferred embodiments the microorganism used in the present invention may be a probiotic bacterium, more preferably a lactic acid bacterium.

In this specification the term 'probiotic bacterium' is defined as covering any non-pathogenic bacterium which, when administered live in adequate amounts, confers a health benefit on the host. These probiotic strains generally have the ability to survive the passage through the upper part of the digestive tract. They are non-pathogenic, non-toxic and exercise their beneficial effect on health on the one hand via ecological interactions with the resident flora in the digestive tract, and on the other hand via their ability to influence the immune system in a positive manner via the "GALT" (gut-associated lymphoid tissue). Depending on the definition of probiotics, these bacteria, when given in a sufficient number, have the ability to progress live through the intestine, however they do not cross the intestinal barrier and their primary effects are therefore induced in the lumen and/or the wall of the gastrointestinal tract. They then form part of the resident flora during the administration period. This colonization (or transient colonization) allows the probiotic bacteria to exercise a beneficial effect, such as the repression of potentially pathogenic micro-organisms present in the flora and interactions with the immune system of the intestine.

### FOOD

The blended complex cultures produced by the method of the present invention may be used in the production of for example a foodstuff or feed, such as a dairy product, e.g. cheese or milk based products, a meat based product, a vegetable food product, a probiotic culture or a biopreservation complex culture.

The blended complex cultures may be used as - or in the preparation of - a food. Here, the term "food" is used in a broad sense - and covers food for humans as well as food for animals (i.e. a feed). In a preferred aspect, the food is for human consumption.

In one embodiment the blended complex cultures may be used as starter cultures in the production of food.

The food may be in the form of a solution or as a solid - depending on the use and/or the mode of application and/or the mode of administration.

When used as - or in the preparation of - a food - such as a functional food - the composition may be used in conjunction with one or more of: a nutritionally acceptable carrier, a nutritionally acceptable diluent, a nutritionally acceptable excipient, a nutritionally acceptable adjuvant, a nutritionally active ingredient.

### FOOD INGREDIENT

The blended complex cultures prepared according to the method of the present invention may be used as a food ingredient.

As used herein the term "food ingredient" includes a formulation which is or can be added to functional foods or foodstuffs as a nutritional supplement and/or fibre supplement. The term food ingredient as used here also refers to formulations which can be used at low levels in a wide variety of products that require gelling, texturising, stabilising, suspending, film-forming and structuring, retention of juiciness and improved mouthfeel, without adding viscosity.

The food ingredient may be in the form of a solution or as a solid - depending on the use and/or the mode of application and/or the mode of administration.

### FOOD SUPPLEMENTS

The blended complex cultures prepared according to the method of the present invention may be - or may be added to - food supplements.

### FUNCTIONAL FOODS

The blended complex cultures prepared according to the method of the present invention may be - or may be added to - functional foods.

As used herein, the term "functional food" means food which is capable of providing not only a nutritional effect and/or a taste satisfaction, but is also capable of delivering a further beneficial effect to consumer.

Accordingly, functional foods are ordinary foods that have components or ingredients (such as those described herein) incorporated into them that impart to the food a specific functional - e.g. medical or physiological benefit - other than a purely nutritional effect.

Although there is no legal definition of a functional food, most of the parties with an interest in this area agree that they are foods marketed as having specific health effects. Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

Surveys have suggested that consumers place the most emphasis on functional food claims relating to heart disease. Preventing cancer is another aspect of nutrition which interests consumers a great deal, but interestingly this is the area that consumers feel they can exert least control over. In fact, according to the World Health Organization, at least 35% of cancer cases are diet-related. Furthermore claims relating to osteoporosis, gut health and obesity effects are also key factors that are likely to incite functional food purchase and drive market development.

### FOOD PRODUCTS

The blended complex cultures prepared according to the present invention can be used in the preparation of food products such as one or more of: jams, marmalades, jellies, dairy products (such as milk or cheese), meat products, vegetable based food products, poultry products, fish products and bakery products.

By way of example, the blended complex cultures can be used as ingredients to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, breakfast cereals, and cup noodles, and balanced foods and drinks, cheese cake flavoured filling, cake and doughnut icing, heat stable bakery filling, instant bakery filling creams, filling for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk.

A blended complex culture prepared according to the present invention can further be used as an ingredient in food products such as American cheese sauce, anti-caking agent for grated & shredded cheese, chip dip, cream cheese, dry blended whip topping fat free sour cream, freeze/thaw dairy whipping cream, freeze/thaw stable whipped tipping, low fat natural cheddar cheese, low fat Swiss style yoghurt, aerated frozen desserts, and novelty bars, hard pack ice cream, label friendly, improved economics & indulgence of hard pack ice cream, low fat ice cream: soft serve, barbecue sauce, cheese dip sauce, cottage cheese dressing, dry mix Alfredo sauce, mix cheese sauce, and others.

For certain aspects, preferably the foodstuff is a beverage. By way of example the beverage may be a fermented milk drink.

For certain aspects, preferably the foodstuff is a bakery product - such as sour dough bread, bread, Danish pastry, biscuits or cookies.

The term "dairy product" as used herein is meant to include a medium comprising milk of animal and/or vegetable origin. As milk of animal origin there can be mentioned cow's, sheep's, goat's or buffalo's milk. As milk of vegetable origin there can be mentioned any fermentable substance of vegetable origin which can be used, in particular originating from soybeans, rice or cereals. Still more preferably blended complex cultures produced by the present invention are employed in or in the production of a food product which is a fermented milk or humanized milk.

For certain aspects, preferably the blended complex cultures may be used in connection with yoghurt production, such as fermented yoghurt drink, yoghurt, drinking yoghurt, cheese, fermented cream, fermented milk, milk based desserts and others.

The term "cheese" as used herein may be any kind of cheese and includes, e.g. natural cheese, cheese analogues and processed cheese. The cheese may be obtained by any suitably process known in the art, such as e.g. by enzymatic coagulation of the cheese milk with rennet, or by acid coagulation of the cheese milk with food grade acid or acid produced by lactic acid bacteria growth. In one embodiment, the cheese may be a rennet-curd cheese.

The cheese for which the blended complex culture may be used include all varieties of cheese, such as, e.g. Campesino, Chester, Danbo, Drabant, Herregård, Manchego, Provoline, Saint Paulin, Soft Cheese, Svecia, Talegio, White Cheese, including rennet-curd cheese produced by rennet-coagulation of the cheese curd; ripened cheeses such as Cheddar, Colby, Edam, Muenster, Gruyere, Emmental, Camembert, Parmesan and Romano; fresh cheeses such as Mozzarella and Feta; acid coagulated cheeses such as cream cheese, Neufchatel, Quarg, Cottage Cheese and Queso Blanco; and pasta filata cheese. The cheese may be a pizza cheese in one embodiment.

The blended complex culture produced by the method of the present invention may be added at any time during the conventional cheese-making process. The blended complex culture may be added to the cheese milk or one or more milk fractions, such as skimmed milk, cream, whole milk, buttermilk from production of sweet or acidified butter, whey protein concentrate, and butter or butter oil.

Suitably, the blended complex cultures can be further used as an ingredient in (or a starter culture for) one or more of cheese applications, meat applications, vegetable applications or applications comprising protective cultures.

Advantageously, there are also provided products that have been contacted with the blended complex cultures prepared according to the method of the present invention (and optionally with other components/ingredients), wherein the blended complex cultures is used in an amount to be capable of improving the nutrition and/or health benefits/functionality of the product.

As used herein the term "contacted" refers to the indirect or direct application of the blended complex cultures prepared according to the method of the present invention to the product. Examples of the application methods which may be used, include, but are not limited to, treating the product in a material comprising the blended complex cultures, direct application by mixing the blended complex cultures with the product, spraying the blended complex cultures onto the product surface or dipping the product into a preparation of the blended complex cultures.

Where the product is a foodstuff, the blended complex culture prepared according to the method of the present invention may be admixed with the food. Alternatively, the blended complex cultures may be included in the emulsion or raw food ingredients of a foodstuff. In a further alternative, the blended complex cultures may be applied as a seasoning, glaze, colorant mixture, and the like.

For some applications, it is important that the blended complex culture is made available on or to the surface of a product or foodstuff to be affected/treated. This may allow the blended complex culture to impart one or more of the following favourable characteristics: nutrition and/or health benefits and/or a particular specific functionality.

The blended complex cultures prepared according to the method of the present invention may be applied to intersperse, coat and/or impregnate a product or foodstuff with a controlled amount of a viable microorganism.

Preferably, the blended complex cultures is used to ferment milk or common fermentable sugars (such as sucrose, glucose, lactose and maltose for example), or fortified milk or lactic media with any common fermentable sugar (such as sucrose, glucose, lactose and maltose for example) where the resulting media containing all components of the blended complex cultures can be added as an ingredient to yoghurt milk in suitable concentrations - such as for example in concentrations in the final product which offer a daily dose of 10⁶-10¹⁰ cfu.

For some aspects the blended complex culture prepared according to the present invention may be used as, or in the preparation of, animal feeds, such as livestock feeds, in particular poultry (such as chicken) feed, or pet food.

### PROBIOTIC/PREBIOTIC

For some applications, it is believed that the viable lactic acid microorganisms in the blended complex culture prepared according to the method of the present invention can exert a probiotic culture effect. It is also within the scope of the present invention to add to the blended complex culture of the present invention further probiotics and/or prebiotics.

The term "probiotic culture" as used herein defines live microorganisms (including bacteria or yeasts for example) which, when for example ingested or locally applied in sufficient numbers, beneficially affect the host organism, i.e. by conferring one or more demonstrable health benefits on the host organism. Probiotics may improve the microbial balance in one or more mucosal surfaces. For example, the mucosal surface may be the intestine, the urinary tract, the respiratory tract or the skin. The term "probiotic" as used herein also encompasses live microorganisms that can stimulate the beneficial branches of the immune system and at the same time decrease the inflammatory reactions in a mucosal surface, for example the gut.

Whilst there are no lower or upper limits for probiotic intake, it has been suggested that at least 10⁶-10¹², preferably at least 10⁶-10¹⁰, preferably 10⁸-10⁹, cfu/mL as a daily dose will be effective to achieve the beneficial health effects in a host organism, such as a human. There are also provided prebiotics as other compounds which can be included in a combination along with the blended complex culture. Here, a prebiotic is: "*a non-digestible food ingredient that beneficially affects the host by selectively stimulating the growth and*/*or the activity of one or a limited number of beneficial bacteria*". The prebiotic component of the combination comprising the blended complex culture are characterised with slow fermentation in the large bowel. Such prebiotics can exert a positive effect on the gut flora, specifically in the left side of the colon, an area of the gut which is especially prone to disorders in particular bowel cancer and ulcerative colitis.

Prebiotics are typically non-digestible carbohydrates (oligo- or polysaccharides) or a sugar alcohol which is not degraded or absorbed in the upper digestive tract. Known prebiotics used in commercial products and useful includes inulin (fructo-oligosaccharide, or FOS) and transgalacto-oligosaccharides (GOS or TOS). Other suitable prebiotics include palatinoseoligosaccharide, soybean oligosaccharide, gentiooligosaccharide, xylooligomers, non-degradable starch, lactosaccharose, lactulose, lactitol, maltitol, polydextrose (i.e. Litesse®) or the like. There is also provided the combination of a blended complex culture prepared according to the method of the present invention with a prebiotic. The prebiotic for use in this combination may be one or more of the above list. The prebiotic may be administered (or added to the food) simultaneously with (e.g. in admixture together with or delivered simultaneously by the same or different routes) or sequentially to (e.g. by the same or different routes) the blended complex culture.

### BIOPRESERVATION PRODUCTS

Biopreservation refers to extended storage life and enhanced safety of foods using the microorganisms having anti-microbial activity, such as anti-Listeria and/or anti-mould activities and (or) their antibacterial products. Lactic acid bacteria have a major potential for use in biopreservation because they are safe to consume and during storage they naturally dominate the microflora of many foods. In milk, brined vegetables, many cereal products and meats with added carbohydrates, the growth of lactic acid bacteria produces a new food product. In raw meats and fish that are chill stored under vacuum or in an environment with elevated carbon dioxide concentration, the lactic acid bacteria become the dominant population and preserve the meat with a hidden fermentation. The same applies to processed meats provided that the lactic acid bacteria survive the heat treatment or they are inoculated onto the product after heat treatment.

In one aspect the blended complex cultures prepared according to the method of the present invention are used in (or in the production of) biopreservation compositions or foods which display an extended storage life and enhanced safety due to the natural microflora and/or their antibacterial products.

### MEAT BASED FOOD PRODUCT

A meat based food product is any product based on meat.

The meat based food product is suitable for human and/or animal consumption as a food and/or a feed. In one embodiment the meat based food product is a feed product for feeding animals, such as for example a pet food product. In another embodiment the meat based food product is a food product for humans.

A meat based food product may comprise non-meat ingredients such as for example water, salt, flour, milk protein, vegetable protein, starch, hydrolysed protein, phosphate, acid, spices, colouring agents and/or texturising agents.

A meat based food product preferably comprises between 5-90% (weight/weight) meat. In some embodiments the meat based food product may comprise at least 30% (weight/weight) meat, such as at least 50%, at least 60% or at least 70% meat.

In some embodiments the meat based food product is a cooked meat, such as ham, loin, picnic shoulder, bacon and/or pork belly for example.

The meat based food product may be one or more of the following:
Dry or semi-dry cured meats - such as fermented products, dry-cured and fermented with starter cultures, for example dry sausages, salami, pepperoni and dry ham;
Emulsified meat products (e.g. for cold or hot consumption), such as mortadella, frankfurter, luncheon meat and pate;
Fish and seafood, such as shrimps, salmon, reformulated fish products, frozen cold-packed fish;
Fresh meat muscle, such as whole injected meat muscle, for example loin, shoulder ham, marinated meat;
Ground and/or restructured fresh meat - or reformulated meat, such as upgraded cut-away meat by cold setting gel or binding, for example raw, uncooked loin chops, steaks, roasts, fresh sausages, beef burgers, meat balls, pelmeni;
Poultry products - such as chicken or turkey breasts or reformulated poultry, e.g. chicken nuggets and/or chicken sausages;
Retorted products - autoclaved meat products, for example picnic ham, luncheon meat, emulsified products.

In one embodiment the meat based food product is a processed meat product, such as for example a sausage, bologna, meat loaf, comminuted meat product, ground meat, bacon, polony, salami or pate.

A processed meat product may be for example an emulsified meat product, manufactured from a meat based emulsion, such as for example mortadella, bologna, pepperoni, liver sausage, chicken sausage, wiener, frankfurter, luncheon meat, meat pate.

The meat based emulsion may be cooked, sterilised or baked, e.g. in a baking form or after being filled into a casing of for example plastic, collagen, cellulose or a natural casing. A processed meat product may also be a restructured meat product, such as for example restructured ham. A meat product of the invention may undergo processing steps such as for example salting, e.g. dry salting; curing, e.g. brine curing; drying; smoking; fermentation; cooking; canning; retorting; slicing and/or shredding.

In one embodiment the meat may be minced meat.

In another embodiment the food product may be an emulsified meat product.

### MEAT

The term "meat" as used herein means any kind of tissue derived from any kind of animal.

The term meat as used herein may be tissue comprising muscle fibres derived from an animal. The meat may be an animal muscle, for example a whole animal muscle or pieces cut from an animal muscle.

In another embodiment the meat may comprise inner organs of an animal, such as heart, liver, kidney, spleen, thymus and brain for example.

The term meat encompasses meat which is ground, minced or cut into smaller pieces by any other appropriate method known in the art.

The meat may be derived from any kind of animal, such as from cow, pig, lamb, sheep, goat, chicken, turkey, ostrich, pheasant, deer, elk, reindeer, buffalo, bison, antelope, camel, kangaroo; any kind of fish e.g. sprat, cod, haddock, tuna, sea ee1, salmon, herring, sardine, mackerel, horse mackerel, saury, round herring, Pollack, flatfish, anchovy, pilchard, blue whiting, pacific whiting, trout, catfish, bass, capelin, marlin, red snapper, Norway pout and/or hake; any kind of shellfish, e.g. clam, mussel, scallop, cockle, periwinkle, snail, oyster, shrimp, lobster, langoustine, crab, crayfish, cuttlefish, squid, and/or octopus.

In one embodiment the meat is beef, pork, chicken, lamb and/or turkey.

### VEGETABLE BASED FOOD PRODUCT

The vegetable based product as taught herein may be any vegetable. Suitably the vegetable based food product as taught herein may be a fermented vegetable product, a brined vegetable, or a pickled vegetable product.

In one embodiment the vegetable based food product as taught herein may be a beverage, for example a beverage containing soya such as a soya vegetable drink. Suitably the vegetable based food product as taught herein may be a fermented vegetable product such as a sauerkraut fermentation, pickles from fresh, green cucumbers, fermented mixed vegetables or any fermented plant or legumes that can be for example onion, celery, beet, lettuce, spinach, broccoli, cauliflower, mushroom, potatoes, radish, cabbage, peas. It can also be silage.

Suitably the vegetable based food product as taught herein may be bean based, such as cheonggukjang, doenjang, miso, natto, soy sauce, stinky tofu, tempeh for example.

Suitably the vegetable based food product as taught herein may be grain based. In some embodiments the vegetable based food product may be a batter made from rice and lentil (*Vigna mungo*) prepared and fermented for baking Idlis and Dosas, amazake, beer, bread, choujiu, gamju, injera, makgeolli, murri, ogi, sake, sikhye, sourdough, rice wine, Malt whisky, grain whisky, Vodka, batter.

The invention will now be described, by way of example only, with reference to the following Figures and Examples.

### EXAMPLES

### MATERIALS AND METHODS

### 1. Processing of the cultivated portions (building blocks)

In a pre-study several physical and chemical parameters were identified which specifically influence the growth of the different microorganisms (with or without the same functionality) in complex dairy cultures.

Each processing of the targeted microorganism started taking at least two portions of a complex culture. The fermentations were done in 5- liter scale using Labfors 2 fermenters (Infors AG, Bottmingen CH) by applying the selected parameter(s). References:
- Adamberg, K., S. Kask, T. M. Laht, and T. Paalme. 2003. The effect of temperature and pH on the growth of lactic acid bacteria: a pH-auxostat study. International Journal of Food Microbiology 85:171-183.
- Boquien, C. Y., G. Corrieu, and M. J. Desmazeaud. 1988. Effect of Fermentation Conditions on Growth of Streptococcus cremoris AM2 and Leuconostoc lactis CNRZ 1091 in Pure and Mixed Cultures 1. Appl. Environ. Microbiol 54:2527-2531.
- Kimoto, H., M. Nomura, and I. Suzuki. 1999. Growth energetics of Lactococcus lactis subsp. lactis biovar diacetilactis in cometabolism of citrate and glucose. International Dairy Journal 9:857-863.
- Starrenburg, M. J. and J. Hugenholtz. 1991. Citrate Fermentation by Lactococcus and Leuconostoc spp. 1. Appl. Environ. Microbiol 57:3535-3540.
- Zurera-Cosano, G., R. M. Garcia-Gimeno, R. Rodriguez-Perez, and C. Hervas-Martinez. 2006. Performance of response surface model for prediction of Leuconostoc mesenteroides growth parameters under different experimental conditions. Food Control 17:429-438.

### 2. Determination of the ratio of the microorganisms in a mesophilic complex culture

### 2.1. Cell count on calcium-citrate agar

This method evaluates the ratio of three different microorganisms (i.e. genera, species, subspecies or biovarieties) in mesophilic complex cultures by agar-plating. The agar medium, containing insoluble Ca-citrate, allows the differentiation between the citrate-fermenters (*L. lactis* subsp. *lactis* biovariety *diacetilactis* and *Leuconostoc pseudomesenteroides*/*Leuconostoc mesenteroides* subsp. *cremoris*) and the non-citrate-fermenters (*L. lactis* subsp. *lactis*/ *L. lactis* subsp. *cremoris).* Citrate-fermenters are recognised by the formation of a cleared zone around the colonies. Within the group of citrate-fermenters the *L. lactis* subsp. *lactis* biovariety *diacetilactis* can be distinguished from the genus *Leuconostoc* by the ability of the latter to split the lactose analogue 5-bromo-4-chloro-3-indolyl-β-D-galactopyranoside (XGal) forming blue colonies.

### 2.2. Quantitative Real-Time qPCR

In addition to the calcium-citrate-agar test, the ratio of three different microorganisms (i.e. genera, species, subspecies or biovarieties) in the fermented product can be measured with a published Real-Time fourplex qPCR assay (Friedrich and Lenke, 2006).

The Multiplex PCR reaction contained PCR buffer, 5 mM MgCl2, 0.8 µM of dNTPs, 2.5 U of HotStar Taq Polymerase (QIAGEN, Hilden, Germany) and suitable primers. Four microliters of template DNA of 1 ng/µL is added to the reaction of 16 µL.

The Multiplex PCR is carried out on a Rotor-Gene model 3000 quantitative PCR cycler (Corbett Research). Fluorescence data is acquired at the end of each elongation step in the four channels FAM, JOE, ROX and Cy5. Reactions are performed in duplicates. For the calculation of relative proportions of each of the taxa relative to DNA encoding bacterial 16SrDNA, the formula of Pfaffl (2001) is applied. Additionally, a qPCR assay for *L. lactis* subsp. *lactis* biovar *diacetilactis* is applied targeting *citF.* The relative proportion is calculated by measuring DNA encoding for *citF* relative to DNA encoding for bacterial *16SrDNA.*

### 2.3. DNA-microarray

Oligonucleotide probes based on *ldh* (lactate dehydrogenase), *celB* (IIc component of the cellobiose-specific Phosphotranferase sytem) and *16SrDNA* sequences are designed to identify the three different microorganisms (i.e. genera, species, subspecies, biovarieties) within the culture on genus, species, subspecies and biovariety level.

The probes are designed by using the software package Allele ID (Allele ID 5.0; Premier Biosoft International) and are synthesized and modified with C6-amino linkages by Metabion, Germany. All pipetting of oligonucleotides is carried out by using an epmotion 5075 liquid handling system (Eppendorf). DNA microarrays are produced using SMP3 pins (Telechem) on a BioOdyssey™ Calligrapher™ MiniArrayer (BioRad).

Oligonucleotides are deposited as 30 µM diameter spots in 2X Spot Buffer (QInstruments) on Epoxy coated glass slides (EBN European Biotech Network) using the robotic arrayer. Relative humidity is kept constant at 50% and temperature is maintained at 20°C throughout spotting.

Printed Epoxy coated slides are incubated at 30 % humidity over night in the dark to allow efficient cross-linking.

The microarray slides are scanned using GenePix 4000 (Axon Instruments Inc.) with an excitation wavelength of 532 nm (Cy3) and 635 nm (Cy5). For automatic spot detection and signal quantification, the image analysis software GenePix Pro 3.0, Axon Instruments Inc. is used.

### References:

- Nickels, Ch. and Leesment, H. 1964. Method for the differentiation and quantitative determination of starter bacteria. Milchwissenschaft 19. 374-378
- Vogensen, F.K., Karst, T., Larsen, J.J., Kringelum, B., Ellejaer, D., Waagner-Nielsen, E.,1987. Improved direct differentiation between Leuconostoc cremoris, Streptococcus lactis subsp. diacetilactis, and Streptococcus cremoris / Streptococcus lactis on agar. Milchwissenschaft 42 (10), 646 - 648.

### 3. Determination of the bacterial strain diversity and the functionality of the complex cultures and of the generated cultivated portions

### 3.1 DNA-Fingerprinting

DNA-fingerprinting is done on whole cultures and on the base of bacterial strain isolates. Bacterial strains are isolated from serial dilution plates on calcium-citrate agar and incubated (16-18h, 30°C) in modified Elliker broth of the following composition:

| **Ingredients** | **g/L** |
|---|---|
| 1. Peptone | 20.0 g |
| 2. Yeast extract | 5.0 g |
| 3. Gelatine | 2.5 g |
| 4. Glucose, *waterfree* | 5.0 g |
| 5. Lactose EP | 5.0 g |
| 6. Saccharose | 5.0 g |
| 7. Sodium chloride | 4.0 g |
| 8. Sodium acetate x 3 H2O | 1.5 g |
| 9. L (+) ascorbic acid | 0.5 g |
| 10. Tween 80 | 0.5 g |
| 11. Sodium glycerophosphate hydrate | 19.0g |
| target pH before sterilization | 7.20 - 7.30 |
| target pH after sterilization: | 6.70 - 6.90 |

After incubation the DNA is extracted. For DNA extraction using the DNeasy™ Tissue Kit cell pellets are resuspended in 180 µL lysis buffer (20 mM Tris-HCl, pH 8.0, 2 mM EDTA, pH 8.0, 1.2 % Triton X-100, 20 mg/mL lysozyme, 100 U/ml Mutanolysin) and incubated for 30 min at 37°C. Afterwards, the DNeasy™ Tissue Kit (Qiagen) is applied according to the guidelines of the manufacturer to isolate DNA. The DNA of whole cultures is extracted without prior cultivation steps. The DNA is submitted to fingerprinting analysis by using specific restriction endonucleases. Fluorescence markers allow the laser-based detection of fragments on the capillary sequencer CEQ8000. Data analysis is performed using the software CEQ 8000, version 9.0.25.

### 3. 2. DNA-micro array

For the selection of suitable cultures and the confirmation of functionality after the processing of the bacterial groups, the DNA-microarray is applied. A set of genes encoding for different functionalities of *Lactococci* and *Leuconostoc* is selected for the *in silico* design of hybridisation probes. These included genes encoding aroma-forming proteins (such as esterases, aminotransferases, enzymes of citrate metabolism etc.), genes related to texture formation in *Lactococci* (*epsA*, *epsB*, *epsC*, *epsX* etc.), genes related to phage resistance (all known *abi* genes, adsorption and injection inhibition, restriction modification systems), and genes encoding bacteriocins.

The probes are designed by using the software package Allele ID (Allele ID 5.0; Premier Biosoft International) and are synthesized and modified with C6-amino linkages by Metabion, Germany. All pipetting of oligonucleotides is carried out by using an epmotion 5075 liquid handling system (Eppendorf). DNA microarrays are produced using SMP3 pins (Telechem) on a BioOdyssey™ Calligrapher™ MiniArrayer (BioRad).

Oligonucleotides are deposited as 30 µM diameter spots in 2X Spot Buffer (QInstruments) on Epoxy coated glass slides (EBN European Biotech Network) using the robotic arrayer. Relative humidity is kept constant at 50% and temperature is maintained at 20°C throughout spotting.

Printed Epoxy coated slides are incubated at 30 % humidity over night in the dark to allow efficient cross-linking.

The microarray slides are scanned using GenePix 4000 (Axon Instruments Inc.) with an excitation wavelength of 532 nm (Cy3) and 635 nm (Cy5). For automatic spot detection and signal quantification, the image analysis software GenePix Pro 3.0, Axon Instruments Inc. is used.

### 3.3 Acidification curves using the CINAC system

In addition, the acidification properties of the cultivated portions are measured in 9% reconstituted skim milk using the CINAC-system, a computer-controlled device to record and evaluate the pH in definite steps.

### References:

- Bolotin et al. (2001) The Complete Genome Sequence of the Lactic Acid Bacterium Lactococcus lactis ssp. lactis IL1403. Genome Res 11, 731-753
- Calevro et al. (2004) Assessment of 35mer amino-modified oligonucleotide based microarray with bacterial samples. J Microbiol Methods 57, 207-218
- Denef et al. (2003) Validation of a more sensitive method for using spotted oligonucleotide DNA microarrays for functional genomics studies on bacterial communities. Environ Microbiol 5, 933-943
- Peplies et al. (2002) Optimization Strategies for DNA Microarray-Based Detection of Bacteria with 16S rRNA-Targeting Oligonucleotide Probes. Appl Environ Microbiol 69, 1397-1407

### EXAMPLE 1: Identification of the main critical parameters for the selective enrichment of Lactococcus lactis (acidifier), L. lactis subsp. lactis biov. diacetilactis and Leuconostoc in PROBAT 505™.

For the selective enrichment of *Lactococcus lactis* (acidifier), *L. lactis* subsp. *lactis* biov. *diacetilactis* and *Leuconostoc* in the three cultivated portions (building blocks) seven different parameters (temperature, pH, aeration, supplementation of the fermentation medium with either citrate, acetate, gluthatione or cysteine) were applied separately to portions of PROBAT 505™ (a mesophilic complex culture available from Danisco A/S). The pre-screening of the parameters was done in a fedbatch-pro® modular system (DASGIP) using a bi-substrate batch medium, an initial enzymatic treatment may be applied. The medium composition is given in table 1.

**Table 1: Medium composition of the basic fermentation medium**

| Ingredient | Amount (in the range of kg / 1000 L) |
|---|---|
| Water | 900 - 950 |
| Skim milk powder | 30 - 40 |
| Yeast extract | 10 - 20 |
| Lactose | 10 - 20 |
| Glucose | 30 - 40 |
| Tween 80 | 0.1 - 1 |
| Manganese(II)-sulfate-monohydrate | 0.001 - 0.01 |

Table 2 shows the mean values and the standard deviation (Stdev) of PROBAT 505™ under normal conditions (pH_{w}=6.0; Temperature=30°C; no aeration; bi-substrate batch with lactose and glucose) in five-liter scale based on 5 fermentations.

**Table 2: Typical ratio of Lactococcus lactis (acidifier), L. lactis subsp. lactis biov. diacetilactis and Leuconostoc tin PROBAT 505™ in five-liter scale**

| | Cell count (CFU/ml Calcium-citrate agar) | | | | Percentage of CFU count (%) | | |
|---|---|---|---|---|---|---|---|
| | *Lc. lactis* acidifier | *Leuconostoc* | *Lc.l.l. diac.* | Total CFU | *Lc. lactis* acidifier | *Leuconostoc* | *Lc.l.l. dicac.* |
| Mean val. | 1.47E+09 | 1.44E+07 | 1.22E+08 | 1.60E+09 | 91.5 | 0.9 | 7.6 |
| Stdev | 5.08E+08 | 6.93E+06 | 9.81E+08 | 5.98E+08 | 34.6 | 48.0 | 80.4 |

### 1. Parameter: Fermentation temperature

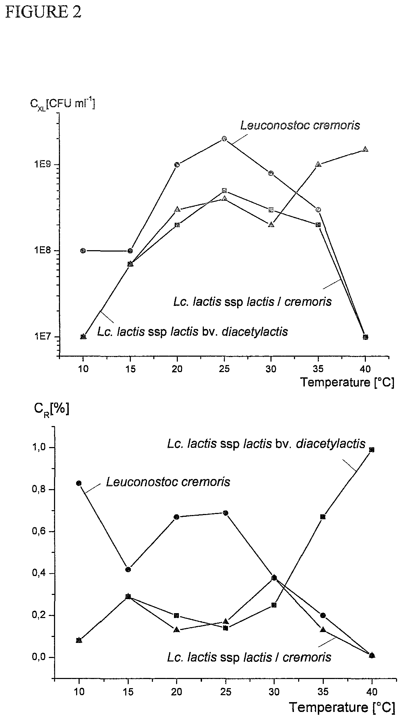

Fermentations of PROBAT 505™ were done at 15, 20, 25, 30, 35 and 40 °C with a controlled pH of 6.0. All fermentations were performed in duplicates. The ratio of *Lactococcus lactis* (acidifier), *L. lactis* subsp. *lactis* biov. *diacetilactis* and *Leuconostoc* was measured on calcium-citrate agar (the results are shown in Figure 2).

The following temperature optima were identified:
- *Lc. lactis* sub. *lactis* / *cremoris* : *25-30 °C* The growth stops at temperatures below 15 °C.
- *L. lactis* subsp. *lactis* biov. *diacetilactis:* 35-40 °C The growth stops at temperatures below 15 °C.
- *Leuconostoc* : 22-25 °C
The growth stops above 35°C. The members of this genus are able to grow at temperatures down to 4°C.

### 2. Parameter: pH during the fermentation

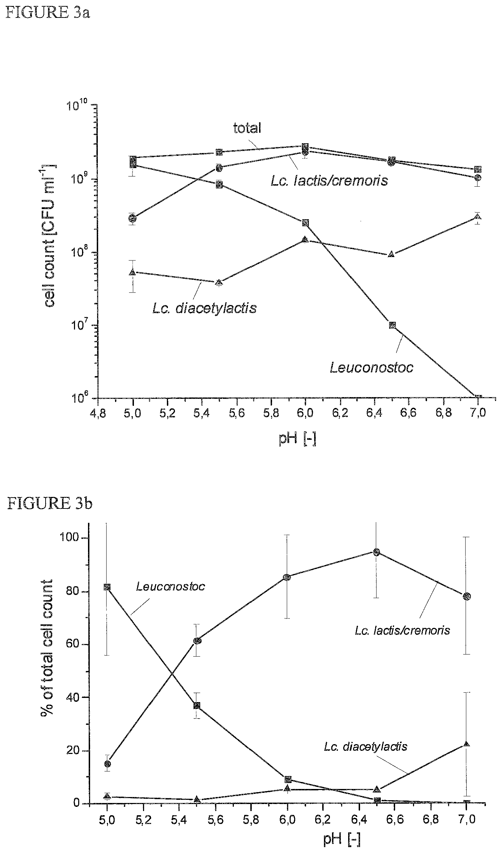

Fermentations of PROBAT 505™ were done at different pH control values (set points; pH_{w} = 4.8 to 7.0). The ratio of the different *Lactococcus lactis* (acidifier), *L. lactis* subsp. *lactis* biov. *diacetilactis* and *Leuconostoc* was measured on calcium-citrate- agar (the results are shown in Figure 3).

The growth of *Lc. lactis (acidifier)* was suppressed at a low pH set point (< 6.0), having its optimum at pHw=6.0. The pH showed a significant impact on the growth of *Leuconostoc.* The highest cell count (1.5x10E9 cfu/mL) of *Leuconostoc* was found at pH=5.0 and decreased with increasing pH to reach its lowest number at pH 7.0 (1x10E6 cfu/mL).

The cell count of *L. lactis* subsp. *lactis* biov. *diacetilactis* increased slightly with increasing pH from 5x10E7 at pHw=5.0 to 3x10E8 at pHw=7.0.

### 3. Parameter: Supplementation of the fermentation medium with different compounds

Different compounds were tested for their effect on the growth of ***Lactococcus lactis* (acidifier), *L. lactis* subsp. *lactis* biov. *diacetilactis* and *Leuconostoc*** t The tested compounds and the concentrations are given in Table 3.

**Table 3: Type and concentration of the tested compounds**

| Compound | Concentration |
|---|---|
| 4-Aminobenzoic acid (PABA) | 70 mg/L |
| L-gluthatione | 1 g/L |
| α-tocopherol | 1 g/L |
| Ascorbic acid | 1 g/L |
| Cysteine | 0.5 g/L |
| Formic acid | 1 g/L |
| Lipoic acid | 0.1 g/L |
| Thymidine triphosphate (TPP) | 0.3 g/L |
| Acetic acid | 7 g/L |
| Citric acid | 13 g/L |
| Inosine + guanosine | 0.3 g/L |

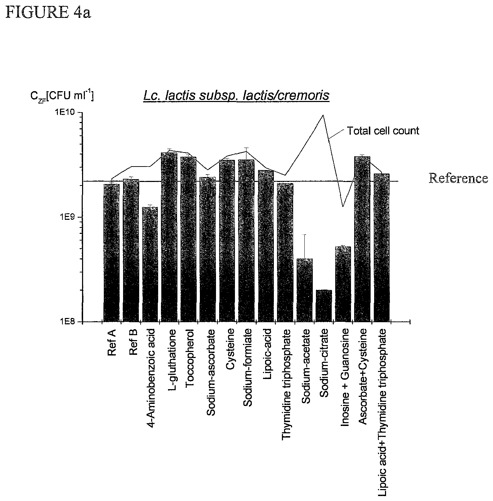

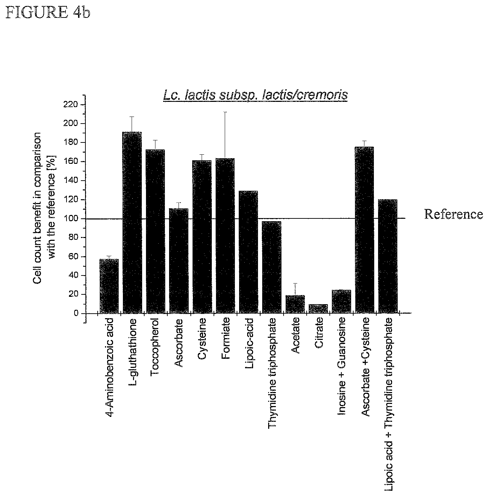

Figure 4 shows the effect of the different compounds on the growth of the acidifier (*Lc. lactis* subsp. *lactis* and *Lactococcos lactis* subsp. *cremoris*).

Glutathione, toccopherol, cysteine and formate promoted the growth of *Lactococcus lactis* (acidifier) in PROBAT 505™ whereas acetate and citrate had an inhibitory effect on this species.

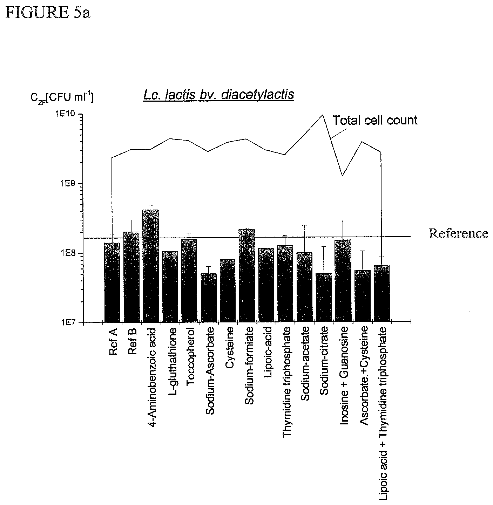

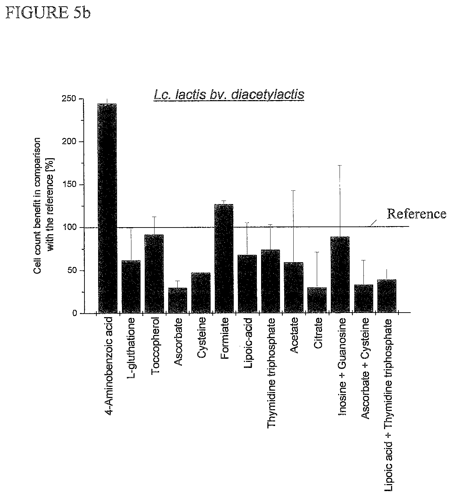

Figure 5 shows the effect of the different compounds on the growth of the aroma producer *Lc. lactis* subsp. *lactis* biovariety *diacetilactis.*

Except for 4-Aminobenzoic acid (PABA) none of the other compounds had a strong positive effect on the growth of *L. lactis* subsp. *lactis* biov. *diacetilactis.* By the addition of 0.7 g/L of PABA to the fermentation medium the cell count increased by 200 to 250 %.

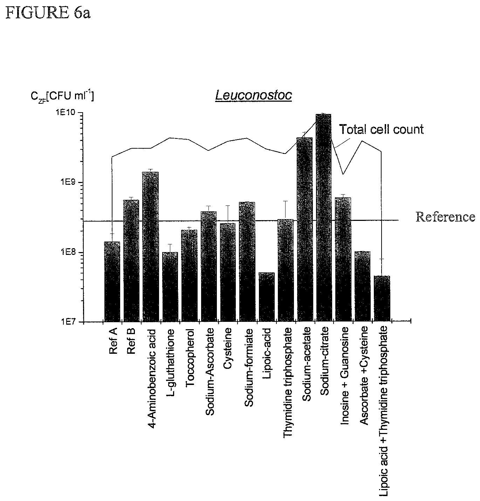

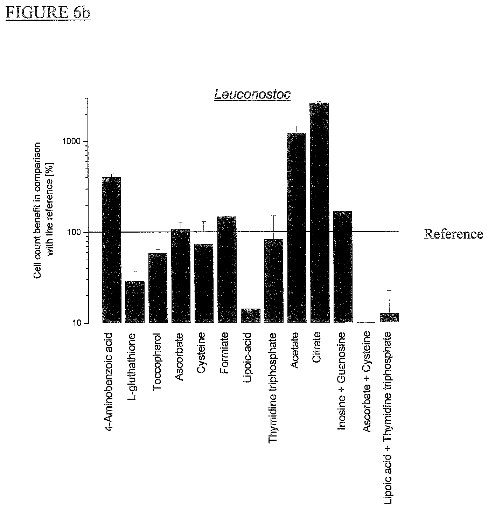

Figure 6 shows the effect of the different compounds on the growth of the aroma producer *Leuconostoc* ssp.

4-Aminobenzoic acid (PABA) and especially the organic acids citrate and acetate promoted the growth of *Leuconostoc* in PROBAT 505™.

### 4. Parameter: Influence on aeration during the fermentation

The aeration was 0.5 vvm (pO_{2W} = 50 % or 80 %) and was controlled by the stirrer. Results are presented in Table 4. Oxygen showed a strong impact on the CFU counts. At 50 % pO2 the cell counts of the *Lc. Lactis* (acidifier) were halved, whereas the CFU of the *Leuconostoc* were doubled.

The CFU of the *Lc. lactis* subsp. *lactis* biov. *diacetilactis* remained at the same level.

**Table 4: Influence of aeration on the ratio of Lactococcus lactis (acidifier), L. lactis subsp. lactis biov. diacetilactis and Leuconostoc**

| % pO2 | Cell count (CFU/ml Calcium-citrate agar) | | | | Percentage of CFU count (%) | | |
|---|---|---|---|---|---|---|---|
| | *Lc. lactis* acidifier | *Leuconostoc* | *Lc.l.l. diac.* | Total CFU | *Lc. lactis* acidifier | *Leuconostoc* | *Lc.l.l. diac.* |
| 0% | 1.66E+09 | 7.46E+07 | 1.29E+08 | 1.86E+09 | 90.2 | 3.3 | 6.5 |
| 50% | 3.15E+08 | 3.65E+08 | 1.23E+08 | 8.03E+08 | 39.2 | 45.5 | 15.3 |
| 80% | 1.80E+08 | 3.40E+08 | 7.85E+07 | 5.99E+08 | 30.1 | 56.8 | 13.1 |

### EXAMPLE 2: Processing of the cultivated portions of the initial complex culture under conditions which enrich each portion in one or more genus, species, subspecies, biovarieties), strains or group of strains of microorganisms.

For the processing of the cultivated portions the identified parameters were applied in combination to portions of the complex culture PROBAT 505™. Each cultivated portion represents a building block of the initial culture. All tests were done in five-liter scale in Prop 3 medium (Table 1). A 3-step-fermentation was done, the selective parameter were applied in the main fermentation only. For a selective enrichment achieving >90% purity of the targeted microorganism, at least two of the identified parameters were applied in combination in the fermentation process.

### Processing of the Lc. lactis (acidifier) portion (building block)

### Parameters for the Lc. Lactis (acidifier) portion: pHw=6.0 Tw = 30°C

The parameters for the processing of the *Lc. Lactis* (acidifier) portion are consistent with the standard process (Table 2). Further fermentations applying these conditions confirmed the suitability of these parameters for the selective enrichment of the acidifier (*Lc. lactis*). It was possible to achieve > 90 % *Lc. lactis* (acidifier) in the cultivated portion.

Table 5 shows the cell count numbers and ratios of *Lactococcus lactis* (acidifier), *L. lactis* subsp. *lactis* biov. *diacetilactis* and *Leuconostoc* of 3 fermentations after application of the *Lc. lactis* (acidifier) parameters at the fermentate step.

**Table 5: Ratio of the microorganisms in the cultivated portions after application of the Lc. lactis (acidifier) parameter**

| | Cell count (CFU/ml calcium-citrate agar) | | | | Percentage of CFU count (%) | | |
|---|---|---|---|---|---|---|---|
| | *Lc. lactis* acidifier | *Leuconostoc* | *Lc.l.l. diac.* | Total CFU | *Lc. lactis* acidifier | *Leuconostoc* | *Lc.l.l. diac.* |
| test 1 | 1.15E+09 | 2.25E+07 | 7.25E+07 | 1.24E+09 | 92.3 | 1.8 | 5.8 |
| test 2 | 2.23E+09 | 2.00E+07 | 2.10E+08 | 2.46E+09 | 90.7 | 0.8 | 8.5 |
| test 3 | 1.10E+09 | 7.50E+06 | 4.40E+07 | 1.15E+09 | 95.5 | 0.6 | 3.8 |

### Processing of the Leuconostoc portion (building block)

### Parameters for the Leuconostoc portion: pH_{w}=5.5 T_{w}=25°C Add compounds: 10 g/L citrate and 8 g/L acetate Fermentation time: 19 - 25h

By applying the parameters a purity of > 90% *Leuconostoc* was achieved in the cultivated portion and the total CFU count of the *Leuconostoc* increased 2-3 fold compared to the conventional fermentation (see Table 6). In total two fermentations were done.

**Table 6: Ratio of the microorganisms in the cultivated portion after application of the Leuconostoc parameters**

| | Cell count (CFU/ml Calcium-citrate agar) | | | | Percentage of CFU count (%) | | |
|---|---|---|---|---|---|---|---|
| | *Lc. lactis* acidifier | *Leuconostoc* | *Lc.l.l. diac.* | Total CFU | *Lc. lactis* acidifier | *Leuconostoc* | *Lc.l.l. diac.* |
| 1. test 1 | 1.50E+08 | 5.00E+09 | 2.00E+08 | 5.35E+09 | 2.8 | 93.5 | 3.7 |
| 2. test 2 | 3.50E+08 | 4.80E+09 | 1.50E+08 | 5.30E+09 | 6.6 | 90.6 | 2.8 |

### Processing of the Lc. lactis ssp. lactis biovar. diacetilactis portion (building block)

### Parameters for the Lc. lactis ssp. lactis biovar. diacetilactis portion:

pH_{w}=6.5 T_{w}=35°C Add compound: 10 g/L citrate

By applying the parameters a purity of >90% *Lc. lactis* ssp. *lactis* biovar. *diacetilactis* was achieved in the cultivated portion and the total CFU count of the *Lc. lactis* ssp. *lactis* biovar. *diacetilactis* increased 2-3 fold compared to the conventional fermentation (see Table 7). In total two fermentations were done.

**Table 7: Ratio of the microorganisms in the cultivated portion after application of the Lc. lactis ssp. lactis biovar. diacetilactis parameters**

| | Cell count (CFU/ml Calcium-citrate agar) | | | | Percentage of CFU count (%) | | |
|---|---|---|---|---|---|---|---|
| | *Lc. lactis* acidifier | *Leuconostoc* | *Lc.l.l. diac.* | Total CFU | *Lc. lactis* acidifier | *Leuconostoc* | *Lc.l.l. diac.* |
| 1. test 1 | 2.50E+08 | 1.50E+08 | 4.80E+09 | 5.20E+09 | 4.8 | 2.9 | 92.3 |
| 2. test 2 | 2,.10E+08 | < 1E+08 | 3.60E+09 | 3.81E+09 | 5.5 | < 1 | 94.5 |

Table 8 summarizes potential critical parameters for the selective enrichment of *Lactococcus lactis* (acidifier), *L. lactis* subsp. *lactis* biov. *diacetilactis* and *Leuconostoc* with the main critical parameters in bold.

**Table 8: Critical parameters for the selective enrichment of Lactococcus lactis (acidifier), L. lactis subsp. lactis biov. diacetilactis and Leuconostoc**

| Parameter | *Leuconostoc* | *L.l.l. diacetilactis* | *L. lactis* (*acidifier*) |
|---|---|---|---|
| **pH** | 5.5 | 6.5 | 6.0 |
| **Temperature** | 25°C | 35°C | 30°C |
| Aeration | | | |
| pO2 50% | Positive effect | No effect | Reduction of growth |
| pO2 80% | Positive effect | Negative effect | Reduction of growth |
| **Citrate** | 10 g/L | 10 g/L | No effect |
| **Acetate** | 8 g/L | No effect | No effect |
| Glutathione | No effect | No effect | Positive effect |
| Cysteine | No effect | No effect | Positive effect |

### EXAMPLE 3: Determination of the functionality in the cultivated portions

The documentation of the abundance of aroma, bacteriocin forming and phage resistance genes in PROBAT 505™ and the cultivated portions thereof can be seen in Tables 9 to 11. It could be shown that the aroma forming genes abundant in PROBAT 505™ were also abundant in at least one of the generated cultivated portions. This was also shown for the bacteriocin forming genes. To date, 5 classes of phage resistance mechanisms exist: i) phage adsorption inhibition, ii) phage injection inhibiting resistances, iii) RM-systems (restriction and modification), iv) abortive infection inhibition - inhibition of phage assembly and v) any other system mediating phage resistance such as CRISPR system. The design of all phage resistance probes is based on the genome sequences of *L. l.* subsp. *cremoris* SK11 and *L. l.* subsp. *lactis* Ll1403. Therefore, no phage resistance based probes specific for the genus *Leuconostoc* have previously been designed.

Except for three (abiJ, RM-1_Lld_hsdS2, RMN_LlaBIIIRM) all phage resistance genes abundant in PROBAT 505™ were represented by at least one of the functional Lactococcal groups. Phage sensitivity tests (Table 12) showed that the three missing functional genes had no impact on the phage resistance of the cultivated portions. Consequently, there is no strain or functionality reduction within the cultivated portions in comparison to PROBAT 505™.

**Table 9: Aroma forming gene distribution in PROBAT 505™ and in the generated cultivated portions**

| | | | Cultivated portions | | |
|---|---|---|---|---|---|
| gene | coding protein | 505™ | 505D (acidifier) | *Lc.l.l. diacetilactis* | *Leuconostoc* |
| araT | aromatic amino acid aminotransferase | 1 | 1 | 1 | 1 |
| citP | citrate permease | 0 | 0 | 1 | 1 |
| metB2 | cystathionine lyase/synthase | 1 | 1 | 1 | 0 |
| bcaT | branched-chain amino acid amino transferase | 1 | 1 | 1 | 0 |
| estA | tributyrin-, caroxylesterase | 1 | 1 | 1 | 1 |
| butA | butA dehydrogenase/reductase | 1 | 0 | 0 | 1 |
| kdcA | ketoacid decarboxylase | 1 | 0 | 1 | 0 |
| citR | citrate reductase | 1 | 0 | 1 | 0 |

**Table 10: Bacteriocin forming gene distribution in PROBAT 505™ and in the generated cultivated portions**

| | | | Cultivated portions | | |
|---|---|---|---|---|---|
| gene | coding protein | 505™ | 505D (acidifier) | *Lc.l.l. diacetilactis* | *Leuconostoc* |
| icnC | lactococcin A ABC transporter ATP binding and permease protein | 1 | 0 | 1 | 0 |
| bacA | Bacteriocin | 1 | 0 | 1 | 0 |
| icnD | lactococcin A ABC transporter permease protein | 1 | 0 | 1 | 1 |

**Table 11: Phage resistance gene distribution in PROBAT 505™ and in the generated cultivated portions**

| | | | Cultivated portions | |
|---|---|---|---|---|
| Class of phage resistance mechanism | Gene | 505™ | 505D (acidifier) | *Lc.l.l. diacetilactis* |
| I | Ads_EPS 1 | 1 | 0 | 1 |
| I | Ads_EPS 2 | 1 | 1 | 1 |
| II | Inj_PIP_1 | 1 | 1 | 0 |
| II | Inj_PIP_2 | 1 | 1 | 1 |
| III | abiA | 0 | 0 | 0 |
| III | abiB | 1 | 1 | 1 |
| III | abiC | 0 | 0 | 0 |
| III | abiD | 0 | 0 | 0 |
| III | abiD1 | 0 | 0 | 0 |
| III | abiE | 0 | 0 | 0 |
| III | abiF | 0 | 0 | 0 |
| III | abiG | 0 | 0 | 0 |
| III | abiH | 1 | 0 | 1 |
| III | abiI | 0 | 0 | 0 |
| III | abiJ | 1 | 0 | 0 |
| III | abiO | 0 | 0 | 0 |
| III | abiQ | 0 | 0 | 0 |
| III | abiTi | 0 | 0 | 0 |
| III | abiLi | 0 | 0 | 0 |
| III | abiR | 0 | 0 | 0 |
| IV | RM-1_Lla1403_hsdS | 1 | 0 | 1 |
| IV | RM-1_Lld_hsdS2 | 1 | 0 | 0 |
| IV | RM-1_Lld_hsdS3 | 1 | 1 | 0 |
| IV | RM-1_Lld_hsdS4 | 1 | 1 | 1 |
| IV | RM-1_Lld1 | 1 | 1 | 0 |
| IV | RM3_LLaF1 | 0 | 0 | 0 |
| IV | RM2_LIDHIC_R | 1 | 0 | 1 |
| IV | RM2_LlaDIR | 1 | 0 | 1 |
| IV | RM2_LlaB1R | 0 | 0 | 0 |
| IV | RMN_LlaB2R | 1 | 0 | 1 |
| IV | RM2_LlaD2R | 1 | 0 | 1 |
| IV | RMN_LlaBIIIRM | 1 | 0 | 0 |
| IV | RMN_LlaG1RM | 0 | 0 | 0 |

For the assessment whether there would be a loss of phage resistance of the generated cultivated portions compared to PROBAT 505™ a phage sensitivity test was done. PROBAT 505™ and the generated cultivated portions *Lc. lactis* (acidifier) and *Lc. lactis* ssp. *lactis* biov. *diacetilactis* were challenged with 20 different phage samples from the Danisco phage collection. After incubation time a deviation of the pH-value of +0.05 and above in comparison to the control indicates a positive phage reaction. As shown in Table 12 no deviation in the pH value was found for the cultivated portions although minor deviations occurred in PROBAT 505™ demonstrating the virulence of the phages towards PROBAT 505™.

**Table 12: Acidification test for PROBAT 505™ and the generated cultivated portions**

| | | | Cultivated portions | |
|---|---|---|---|---|
| | Phage | PROBAT 505™ | 505D (acidifier) | *Lc.l.l. diacetilactis* |
| Control | - | 4.70 | 4.64 | 5.89 |
| | P677 | 4.77 | 4.63 | 5.34 |
| | P751 | 4.81 | 4.63 | 5.01 |
| | P775 | 4.78 | 4.63 | 4.83 |
| | P806 | 4.77 | 4.64 | 4.80 |
| | P816 | 4.79 | 4.66 | 4.99 |
| | P855 | 4.80 | 4.64 | 5.05 |
| | P1020 | 4.76 | 4.65 | 4.96 |
| | P1060 | 4.78 | 4.64 | 4.88 |
| | P1096 | 4.78 | 4.63 | 4.90 |
| | P1128 | 4.75 | 4.63 | 4.86 |
| | P1154 | 4.76 | 4.64 | 4.80 |
| | P1307 | 4.78 | 4.63 | 4.75 |
| | P1389 | 4.77 | 4.61 | 4.73 |
| | P1423 | 4.74 | 4.62 | 4.81 |
| | P1429 | 4.78 | 4.62 | 4.82 |
| | P1451 | 4.76 | 4.62 | 4.77 |
| | P1456 | 4.78 | 4.62 | 4.73 |
| | P1469 | 4.80 | 4.63 | 4.78 |
| | P1548 | 4.78 | 4.61 | 4.74 |
| | P1588 | 4.78 | 4.65 | 4.76 |

### EXAMPLE 4: Determination of the bacterial strain diversity in the cultivated portions)

For the proof of strain diversity and to detect major strain losses following the cultivation steps DNA-fingerprints of bacterial strains isolated from the initial complex culture and the cultivated portions were analyzed. Because of the comparably low number of analyzed isolates, the number of detectable different bacterial strains is limited. The actual number of different bacterial strains would be much higher.

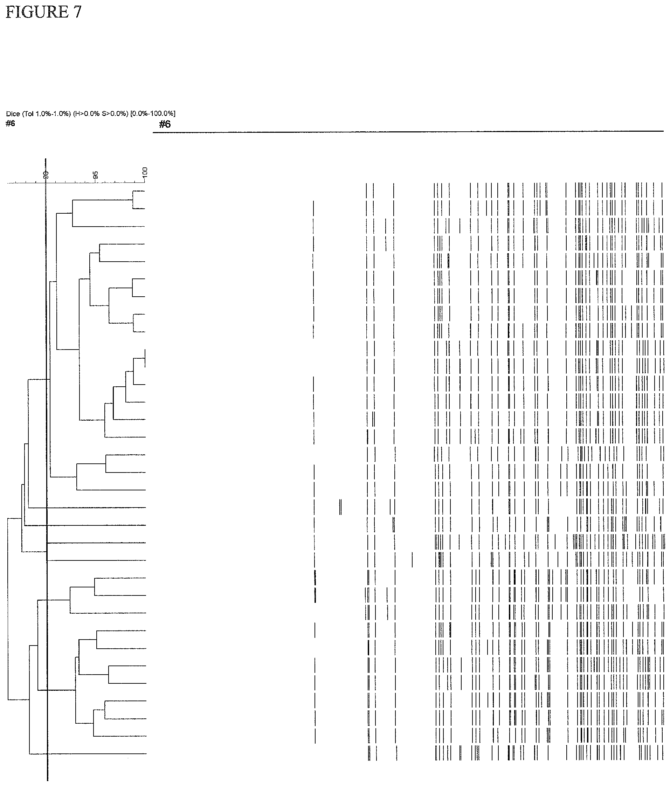

Figure 7 shows a consensus dendrogram of different *Lc. lactis* strain types in a complex culture. One DNA-fingerprint pattern represents one bacterial isolate. In this culture at least 7 different strain types out of 33 analyzed isolates were detected. Bacterial strains showing less than 90% identity, were considered to belong to different strain types.

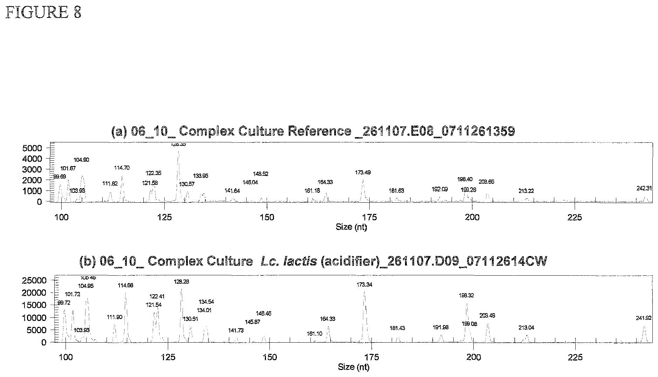

Figure 8 shows the DNA-fingerprint of an initial complex culture and of the cultivated portion *Lc. lactis* (acidifier). The pattern of (a) and (b) are identical, indicating no loss of strain diversity in the cultivated portion.

### EXAMPLE 5: Blending of the produced cultivated portions in a way that the same technological properties of the initial culture were achieved

The produced cultivated portions of PROBAT 505™ were re-blended following the specification of the initial culture PROBAT 505™.

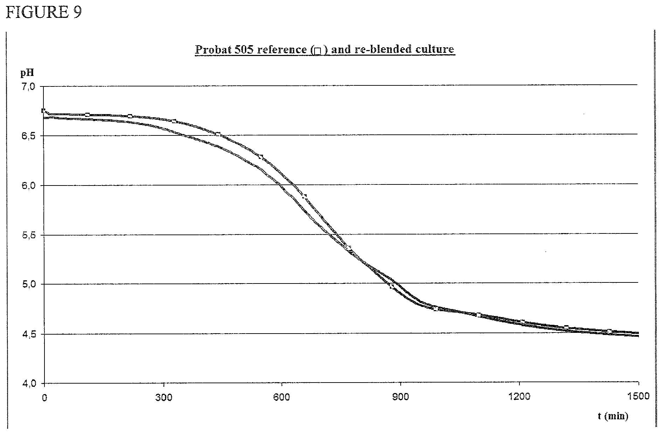

By re-blending of the cultivated portions of PROBAT 505™ the acidifying properties of the initial complex culture PROBAT 505™ were achieved (see Figure 9). The sensorial properties were evaluated by a trained test panel. No major deviation of the re-blended culture compared to the original PROBAT 505™ was detected.

### EXAMPLE 6: Blending of two cultivated portions from two different complex cultures and single bacterial strains to a new complex culture with the same sensorial and acidifying properties as PROBAT 505™ but having a different phage lysotype

In this example the *Lc. lactis* (acidifier) and the *Lc. lactis* ssp. *lactis* biovar. *diacetilactis* portion were obtained from two different complex cultures by applying the same parameters as for the processing of the cultivated portions of PROBAT 505™ (a first complex culture). Additionally single *Leuconostoc* strains were added to the blend. Both portions and the single *Leuconostoc* strains have a different lysotype compared to the corresponding cultivated portions of the PROBAT 505™ culture.

### Processing of the Lc.lactis (acidifier) portion of a second complex culture

For the processing of the *Lc. lactis* (acidifier) portion another (a second) complex culture was selected. Table 13 shows the ratio of the microorganisms of the culture (reference C) before and after the application of the *Lc. lactis* (acidifier) parameters. By application of the *Lc. lactis* (acidifier) parameters to the second complex culture a pure *Lc. lactis* (acidifier) portion was obtained (however it remains a complex culture because of its high strain diversity).

**Table 13: Ratio of the microorganisms in the cultivated portion before (reference C) and after (1. test 1 and 2. test 2) application of the Lc. lactis (acidifier) parameters**

| | Cell count (CFU/ml Calcium-citrate agar) | | | | Percentage of CFU count (%) | | |
|---|---|---|---|---|---|---|---|
| | *Lc. lactis* acidifier | *Leuconostoc* | *Lc.l.l. diac.* | Total CFU | *Lc. lactis* acidifier | *Leuconostoc* | *Lc.l.l. diac.* |
| Reference C | 3.70E+08 | 3.80E+07 | <10E+06 | 4.08E+08 | 90.70% | 9.30% | < 1% |
| 1. test 1 | 4.25E+09 | <10E+06 | <10E+06 | 4.25E+09 | 100% | 0% | 0% |
| 2.test 2 | 5.35E+09 | <10E+06 | <10E+06 | 5.35E+09 | 100% | 0% | 0% |

### Processing of the Lc. lactis ssp. lactis biovar. diacetilactis portion of a third complex culture

For the processing of the *Lc. lactis* ssp. *lactis* biovar. *diacetilactis* portion another (third) complex culture was selected. Table 14 shows the ratio of the microorganisms of the culture (reference D) before and after the application of the *Lc. lactis* ssp. *lactis* biovar. *diacetilactis* parameters. By application of the *Lc. lactis* ssp. *lactis* biovar. *diacetilactis* parameters to this complex culture a *Lc. lactis* ssp. *lactis* biovar. *diacetilactis* portion of > 90% purity was obtained.

**Table 14: Ratio of the microorganisms in the cultivated portion before (reference D) and after (1. test 1) application of the Lc. lactis ssp. lactis biovar. diacetilactis parameters to another complex culture**

| | Cell count (CFU/ml Calcium-citrate agar) | | | | Percentage of CFU count (%) | | |
|---|---|---|---|---|---|---|---|
| | *Lc.lactis* acidifier | *Leuconostoc* | *Lc.l.l. diac.* | Total CFU | *Lc. lactis* acidifier | *Leuconostoc* | *Lc.l.l. diac.* |
| Reference D | 5.60E+07 | 2.50E+06 | 9.6E+07 | 1.55E+08 | 36% | 2% | 62% |
| 1. test 1 | 3.50E+08 | <10E+06 | 4.28E+09 | 4.63E+09 | 7.6% | 0% | 92.4% |

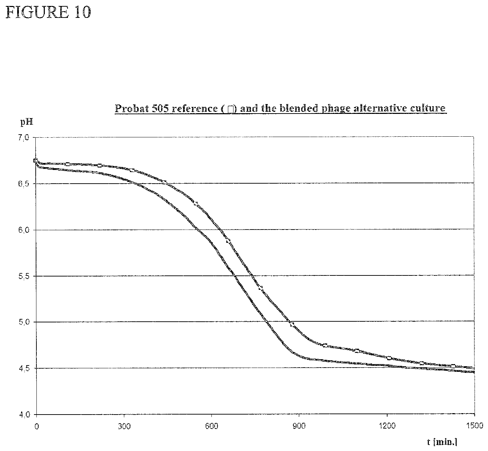

The produced cultivated portions of the second and third complex cultures and the single strains of *Leuconostoc* were blended following the specification of the culture PROBAT505™. As shown in Figure 10 the blended phage-alternative culture was much faster than the complex culture PROBAT 505™. Compared to PROBAT505™ less culture material would be needed to obtain the same properties.

The sensorial properties were evaluated by a trained test panel. The phage-alternative blended complex culture had the same sensory as the PROBAT 505™ reference culture.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

## Claims

1. A method of preparing a blended complex culture of microorganisms comprising:
a) taking at least two portions of an initial complex culture comprising at least 5 different microorganisms;
b) culturing each of the portions under conditions which enrich each portion in one or more microorganisms to obtain a cultivated portion, wherein each portion is cultured under a different culture condition(s) compared with the other portion(s) such that each cultivated portion imparts a different functionality; and
c) formulating a blended complex culture comprising at least one of said cultivated portions, by either c1) mixing or blending at least 2 of the cultivated portions or c2) mixing or blending at least one of the cultivated portions with a different complex culture or cultivated portion thereof.

2. A method according to claim 1 wherein the microorganism is a bacterium.

3. A method according to claim 1 or claim 2 wherein the initial complex culture or the blended complex culture is selected from one or more of the group consisting of: a complex dairy culture, a meat based food product complex culture, a vegetable based food product complex culture, a complex probiotic culture and a complex biopreservation culture.

4. The method according to any one of the preceding claims wherein the conditions for culturing the portions are determined by testing each portion or a part thereof and determining at least one critical parameter for each portion which enables enrichment of a specific microorganism or enables enrichment of a specific mixture of microorganisms, and culturing each portion under a condition(s) which includes said at least one critical parameter.

5. A method according to any one of the preceding claims wherein the method further comprises testing each portion or a part thereof to determine the effect on the microorganism(s) in the portion of one or more parameters selected from the group consisting of: pH; temperature; agitation speed; phosphate salts; aeration; citrate; acetate; glutathione; cysteine; addition of protectants, e.g. used during freezing; cooling time, freezing time, pelleting size, parameters for preservation.

6. A method according to any one of the preceding claims wherein the method further comprises analysing and/or testing i) the initial complex culture, ii) a portion or part thereof, iii) the cultivated portion and/or iv) the blended complex culture to determine one or more of the following: species or strain diversity; genetic relationships between the microorganisms in the portion; DNA-fingerprinting of the microorganism(s) in portion; bacteriophage sensitivity; bacteriophage resistance; lysotype of the microorganim(s); the presence of functional genes which may result in preferred or detrimental functional effect; the functional properties of the portion; bacteriocin producing properties of the portion, rheological properties, aroma, taste, texture, viscosity, probiotic properties, acidification, gas formation, lactose reduction, health benefits (for example production of certain vitamins), production of substances that promote the growth of the culture and interaction between the microorganisms, production of substances that increase the survival of microorganims after freezing and/or freeze drying, production of biopreservative substances, capacity to remove undesirable compounds by enzymatic activity (e.g. production or use of bitter peptides causing an off-flavour).

7. The method according to any one of the preceding claims wherein the one or more microorganisms or mixture of microorganisms in each cultivated portion are different microorganisms or mixtures of microorganisms compared with the other cultivated portion(s).

8. The method according to claim 7, wherein the genus, species, subspecies or biovariety of microorganism or mixture of microorganisms in each cultivated portion is different compared with the other cultivated portion(s).

9. The method according to claim 1 wherein the functionality may be selected from one or more of the group consisting of: probiotic properties, acidification, aroma enhancement. gas formation, texturisation, lactose reduction, nitrate reduction, bacteriophage sensitivity; bacteriophage resistance, health benefits (for example production of certain vitamins), production of substances that promote the growth of the culture and interaction between the microorganisms, production of substances that increase the survival of microorganims after freezing and/or freeze drying, production of biopreservative substances, capacity to remove undesirable compounds by enzymatic activity (e.g. production or use of bitter peptides causing an off-flavour).

10. The method according to any one of the preceding claims wherein the enrichment of one or more microorganism(s) in each portion is such that more than 80% of the microorganim(s) in the cultivated portion is comprised of the enriched microorganism(s).

11. The method according to claim 10, wherein more than 90% or more than 95% of the microorganim(s) in the cultivated portion is comprised of the enriched microorganism(s).

12. The method according to any one of the preceding claims comprising mixing at least 2, at least 3 or at least 4 of the cultivated portions, and optionally at least one microorganism which has not been taken from the initial complex culture and/or a different complex culture or cultivated portion thereof.

13. The method according to claim 12 where said culture of microorganisms which has not been taken from the initial complex culture comprises a bacteriophage resistant bacterium or a bacterium that is not infected by bacteriophage.

14. The method according to any one of the preceding claims comprising admixing at least two of said cultivated portions, preferably admixing all of the cultivated portions obtained from culturing all of the portions taken from the initial complex culture.

15. The method according to any one of the preceding claims wherein the method comprises freezing, drying and/or encapsulating either the cultivated portions (of step b) or the blended complex culture (of step c) or both to produce a frozen, dried and/or encapsulated cultivated portion and/or blended complex culture.

16. The method according to any one of the preceding claims wherein the method comprises adding either the cultivated portions (of step b) or the blended complex culture (of step c) to a food ingredient to produce a food product, such as a dairy product, a meat based food product, a vegetable based food product, a probiotic composition or a biopreservation composition.

17. Use of two or more cultivated portions to manufacture a blended complex culture, each cultivated portion being obtained after culturing two or more portions - from a complex culture - under different culture conditions which enrich each portion in one or more different microorganisms (e.g. bacteria) and such that each cultivated portion imparts a different functionality.

## Patentansprüche

1. Verfahren zur Herstellung einer gemischten komplexen Kultur von Mikroorganismen, das umfasst:
a) Abnehmen von wenigstens zwei Anteilen einer komplexen Ausgangskultur, die wenigstens 5 unterschiedliche Mikroorganismen umfasst;
b) Kultivieren von jedem der Anteile unter Bedingungen, die jeden Anteil bezüglich eines oder mehrerer Mikroorganismen anreichern, um einen kultivierten Anteil zu erhalten, wobei jeder Anteil verglichen mit den/m anderen Anteil(en) unter (einer) unterschiedlichen Kulturbedingung(en) kultiviert wird, sodass jeder kultivierte Anteil eine unterschiedliche Funktionalität verleiht; und
c) Formulieren einer gemischten komplexen Kultur, die wenigstens einen der kultivierten Anteile umfasst, durch entweder c1) Mischen oder Vermengen von wenigstens 2 der kultivierten Anteile oder c2) Mischen oder Vermengen von wenigstens einem der kultivierten Anteile mit einer unterschiedlichen komplexen Kultur oder einem kultivierten Anteil davon.

2. Verfahren nach Anspruch 1, wobei es sich bei dem Mikroorganismus um ein Bakterium handelt.

3. Verfahren nach Anspruch 1 oder 2, wobei die komplexe Ausgangskultur oder die gemischte komplexe Kultur aus einem oder mehreren der Gruppe ausgewählt ist, die aus einer komplexen Milchkultur, einer komplexen Fleischbasiertes-Lebensmittelprodukt-Kultur, einer komplexen Pflanzlich-Basiertes-Lebensmittelprodukt-Kultur, einer komplexen probiotischen Kultur und einer komplexen Biokonservierungskultur besteht.

4. Verfahren nach einem der vorhergehenden Ansprüche, wobei die Bedingungen zum Kultivieren der Anteile bestimmt werden durch Testen jedes Anteils oder eines Teils davon und Bestimmen von wenigstens einem wichtigen Parameter für jeden Anteil, der die Anreicherung eines bestimmten Mikroorganismus ermöglicht oder die Anreicherung eines bestimmten Gemisches von Mikroorganismen ermöglicht, und Kultivieren von jedem Anteil unter (einer) Bedingung(en), die den wenigstens einen wichtigen Parameter umfasst bzw. umfassen.

5. Verfahren nach einem der vorhergehenden Ansprüche, wobei das Verfahren weiter das Testen von jedem Anteil oder eines Teils davon umfasst, um die Wirkung von einem oder mehreren Parametern, die aus der Gruppe ausgewählt sind, die aus pH-Wert, Temperatur, Rührgeschwindigkeit, Phosphatsalzen, Belüftung, Citrat, Acetat, Glutathion, Cystein, Zusatz von Schutzmitteln, die z. B. während des Einfrierens verwendet werden, Abkühlzeit, Gefrierzeit, Pelletgröße, Parametern zur Konservierung besteht, auf den Mikroorganismus bzw. die Mikroorganismen in dem Anteil zu bestimmen.

6. Verfahren nach einem der vorhergehenden Ansprüche, wobei das Verfahren weiter das Analysieren und/oder Testen i) der komplexen Ausgangskultur ii) eines Anteils oder Teils davon, iii) des kultivierten Anteils und/oder iv) der gemischten komplexen Kultur umfasst, um eines oder mehrere des Folgenden zu bestimmen: Art- oder Stammdiversität, genetische Verwandtschaften zwischen den Mikroorganismen in dem Anteil, DNA-Fingerabdruck des Mikroorganismus bzw. der Mikroorganismen in dem Anteil, Bakteriophagenempfindlichkeit, Bakteriophagenresistenz, Lysotyp des Mikroorganismus bzw. der Mikroorganismen, Vorhandensein funktioneller Gene, die zu einer bevorzugten oder schädlichen funktionellen Wirkung führen könnten, die funktionellen Eigenschaften des Anteils, Bakteriocin-produzierende Eigenschaften des Anteils, rheologische Eigenschaften, Aroma, Geschmack, Textur, Viskosität, probiotische Eigenschaften, Ansäuerung, Gasbildung, Lactosereduzierung, Gesundheitsvorteile (zum Beispiel Produktion bestimmter Vitamine), Produktion von Stoffen, die das Wachstum der Kultur und eine Wechselwirkung zwischen Mikroorganismen fördern, Produktion von Stoffen, die das Überleben von Mikroorganismen nach Einfrieren und/oder Gefriertrocknung erhöhen, Produktion von Biokonservierungsstoffen, Fähigkeit, unerwünschte Verbindungen durch enzymatische Aktivität zu entfernen (z. B. Produktion oder Verwendung bitterer Peptide, die einen Off-Flavor verursachen).

7. Verfahren nach einem der vorhergehenden Ansprüche, wobei es sich bei dem einen oder den mehreren Mikroorganismen oder dem Gemisch von Mikroorganismen in jedem kultivierten Anteil verglichen mit dem/n anderen kultivierten Anteil(en) um unterschiedliche Mikroorganismen oder Gemische von Mikroorganismen handelt.

8. Verfahren nach Anspruch 7, wobei die Gattung, Art, Unterart oder Biovarietät des Mikroorganismus oder des Gemisches von Mikroorganismen in jedem kultivierten Anteil verglichen mit dem/n anderen kultivierten Anteil(en) unterschiedlich ist.

9. Verfahren nach Anspruch 1, wobei die Funktionalität aus einer oder mehreren aus der Gruppe ausgewählt sein kann, die aus probiotischen Eigenschaften, Ansäuerung, Aromaverstärkung, Gasbildung, Texturierung, Lactosereduzierung, Nitratreduzierung, Bakteriophagenempfindlichkeit, Bakteriophagenresistenz, Gesundheitsvorteile (zum Beispiel Produktion bestimmter Vitamine), Produktion von Stoffen, die das Wachstum der Kultur und eine Wechselwirkung zwischen den Mikroorganismen fördern, Produktion von Stoffen, die das Überleben von Mikroorganismen nach Einfrieren und/oder Gefriertrocknung erhöhen, Produktion von Biokonservierungsstoffen, der Fähigkeit, unerwünschte Verbindungen durch enzymatische Aktivität zu entfernen (z. B. Produktion oder Verwendung bitterer Peptide, die einen Off-Flavor verursachen), besteht.

10. Verfahren nach einem der vorhergehenden Ansprüche, wobei die Anreicherung von einem Mikroorganismus oder mehreren Mikroorganismen in jedem Anteil derart ist, dass mehr als 80% des Mikroorganismus bzw. der Mikroorganismen in dem kultivierten Anteil aus dem angereicherten Mikroorganismus bzw. den angereicherten Mikroorganismen bestehen.

11. Verfahren nach Anspruch 10, wobei mehr als 90% oder mehr als 95% des Mikroorganismus bzw. der Mikroorganismen in dem kultivierten Anteil aus dem angereicherten Mikroorganismus bzw. den angereicherten Mikroorganismen bestehen.

12. Verfahren nach einem der vorhergehenden Ansprüche, welches das Mischen von wenigstens 2, wenigstens 3 oder wenigstens 4 der kultivierten Anteile und wahlweise wenigstens einem Mikroorganismus umfasst, der nicht der komplexen Ausgangskultur und/oder einer unterschiedlichen komplexen Kultur oder einem kultivierten Teil davon entnommen wurde.

13. Verfahren nach Anspruch 12, wobei die Kultur von Mikroorganismen, die nicht der komplexen Ausgangskultur entnommen wurde, ein bakteriophagenresistentes Bakterium oder ein Bakterium, das nicht von Bakteriophagen infiziert ist, umfasst.

14. Verfahren nach einem der vorhergehenden Ansprüche, welches das Vermischen von wenigstens zwei der kultivierten Anteile, bevorzugt das Vermischen aller kultivierten Anteile umfasst, die durch das Kultivieren aller Anteile erhalten wurden, die der komplexen Ausgangskultur entnommen wurden.

15. Verfahren nach einem der vorhergehenden Ansprüche, wobei das Verfahren Einfrieren, Trocknen und/oder Verkapseln von entweder den kultivierten Anteilen (aus Schritt b) oder der gemischten komplexen Kultur (aus Schritt c) oder beiden umfasst, um eine(n) gefrorene(n), getrocknete(n) und/oder verkapselte(n) kultivierten Anteil und/oder gemischte komplexe Kultur herzustellen.

16. Verfahren nach einem der vorhergehenden Ansprüche, wobei das Verfahren das Zugeben von entweder den kultivierten Anteilen (aus Schritt b) oder der gemischten komplexen Kultur (aus Schritt c) zu einem Lebensmittelinhaltsstoff umfasst, um ein Lebensmittelprodukt herzustellen, wie z. B. ein Milchprodukt, ein fleischbasiertes Lebensmittelprodukt, ein pflanzlich basiertes Lebensmittelprodukt, eine probiotische Zusammensetzung oder eine Biokonservierungszusammensetzung.

17. Verwendung von zwei oder mehreren kultivierten Anteilen zur Herstellung einer gemischten komplexen Kultur, wobei jeder kultivierte Anteil erhalten wird, nachdem zwei oder mehrere Anteile - aus einer komplexen Kultur - unter unterschiedlichen Kulturbedingungen kultiviert wurden, die jeden Anteil bezüglich eines oder mehrerer unterschiedlicher Mikroorganismen (z. B. Bakterien) anreichern, und derart, dass jeder kultivierte Anteil eine unterschiedliche Funktionalität verleiht.

## Revendications

1. Méthode de préparation d'une culture complexe assemblée de microorganismes, comprenant les étapes consistant à :
a) prélever au moins deux portions d'une culture complexe initiale comprenant au moins 5 microorganismes différents ;
b) cultiver chacune des portions dans des conditions qui enrichissent chaque portion en un ou plusieurs microorganismes afin d'obtenir une portion cultivée, où chaque portion est cultivée dans une ou plusieurs conditions de cultures différentes par rapport à la ou les autres portions de sorte que chaque portion cultivée confère une fonctionnalité différente ; et
c) formuler une culture complexe assemblée comprenant au moins l'une desdites portions cultivées, en c1) mélangeant ou assemblant au moins 2 des portions cultivées ou bien c2) mélangeant ou assemblant au moins l'une des portions cultivées avec une culture complexe différente ou une portion cultivée de celle-ci.

2. Méthode selon la revendication 1, où le microorganisme est une bactérie.

3. Méthode selon la revendication 1 ou la revendication 2, où la culture complexe initiale ou la culture complexe assemblée est choisie parmi une ou plusieurs dans le groupe constitué par : une culture laitière complexe, une culture complexe de produit alimentaire à base de viande, une culture complexe de produit alimentaire à base de légumes, une culture probiotique complexe et une culture de bio-conservation complexe.

4. Méthode selon l'une quelconque des revendications précédentes, où les conditions de culture des portions sont déterminées en testant chaque portion ou une partie de celle-ci et en déterminant au moins un paramètre critique pour chaque portion qui permet un enrichissement en un microorganisme spécifique ou permet un enrichissement en un mélange spécifique de microorganismes, et en cultivant chaque portion dans une ou plusieurs conditions qui comportent ledit au moins un paramètre critique.

5. Méthode selon l'une quelconque des revendications précédentes, où la méthode comprend en outre l'étape consistant à tester chaque portion ou une partie de celle-ci afin de déterminer l'effet sur le ou les microorganismes dans la portion d'un ou plusieurs paramètres choisis dans le groupe constitué par : le pH ; la température ; la vitesse d'agitation ; les sels de phosphate ; l'aération ; le citrate ; l'acétate ; le glutathion ; la cystéine ; l'addition de protecteurs, par exemple utilisés lors de la congélation ; le temps de refroidissement, le temps de congélation, la taille de pelletisation, les paramètres de conservation.

6. Méthode selon l'une quelconque des revendications précédentes, où la méthode comprend en outre l'étape consistant à analyser et/ou tester i) la culture complexe initiale, ii) une portion ou une partie de celle-ci, iii) la portion cultivée et/ou iv) la culture complexe assemblée, afin de déterminer un(e) ou plusieurs parmi ce qui suit : la diversité d'espèce ou de souche ; les relations génétiques entre les microorganismes dans la portion ; l'identification génétique du ou des microorganismes dans la portion ; la sensibilité aux bactériophages ; la résistance aux bactériophages ; le lysotype du ou des microorganismes ; la présence de gènes fonctionnels qui peut entraîner un effet fonctionnel préféré ou préjudiciable ; les propriétés fonctionnelles de la portion ; les propriétés de production de bactériocine de la part de la portion, les propriétés rhéologiques, l'arôme, le goût, la texture, la viscosité, les propriétés probiotiques, l'acidification, la formation de gaz, la réduction du lactose, les bénéfices pour la santé (par exemple la production de certaines vitamines), la production de substances qui favorisent la croissance de la culture et l'interaction entre les microorganismes, la production de substances qui augmentent la survie des microorganismes après congélation et/ou lyophilisation, la production de substances bio-conservatrices, la capacité à éliminer des composés non désirés par une activité enzymatique (par exemple la production ou l'utilisation de peptides amers provoquant une saveur passée).

7. Méthode selon l'une quelconque des revendications précédentes, où les un ou plusieurs microorganismes ou le mélange de microorganismes dans chaque portion cultivée sont des microorganismes ou mélanges de microorganismes différents par rapport à l'autre ou aux autres portions cultivées.

8. Méthode selon la revendication 7, où le genre, l'espèce, la sous-espèce ou la bio-variété des microorganismes ou du mélange de microorganismes dans chaque portion cultivée est différent(e) par rapport à l'autre ou aux autres portions cultivées.

9. Méthode selon la revendication 1, où la fonctionnalité peut être choisie parmi un(e) ou plusieurs dans le groupe constitué par : les propriétés probiotiques, l'acidification, l'amélioration de l'arôme, la formation de gaz, la texturisation, la réduction du lactose, la réduction des nitrates, la sensibilité aux bactériophages, la résistance aux bactériophages, les bénéfices pour la santé (par exemple la production de certaines vitamines), la production de substances qui favorisent la croissance de la culture et l'interaction entre les microorganismes, la production de substances qui augmentent la survie des microorganismes après congélation et/ou lyophilisation, la production de substances bio-conservatrices, la capacité à éliminer des composés non désirés par une activité enzymatique (par exemple la production ou l'utilisation de peptides amers provoquant une saveur passée).

10. Méthode selon l'une quelconque des revendications précédentes, où l'enrichissement en un ou plusieurs microorganismes dans chaque portion est tel que plus de 80% du ou des microorganismes dans la portion cultivée sont constitués du ou des microorganismes enrichis.

11. Méthode selon la revendication 10, où plus de 90% ou plus de 95% du ou des microorganismes dans la portion cultivée sont constitués du ou des microorganismes enrichis.

12. Méthode selon l'une quelconque des revendications précédentes, comprenant le mélange d'au moins 2, au moins 3 ou au moins 4 des portions cultivées, et éventuellement d'au moins un microorganisme n'ayant pas été prélevé à partir de la culture complexe initiale et/ou d'une culture complexe différente ou d'une portion cultivée de celle-ci.

13. Méthode selon la revendication 12, où ladite culture de microorganismes n'ayant pas été prélevée à partir de la culture complexe initiale comprend une bactérie résistante aux bactériophages ou une bactérie qui n'est pas infectée par des bactériophages.

14. Méthode selon l'une quelconque des revendications précédentes, comprenant le mélange d'au moins deux desdites portions cultivées, préférablement le mélange de l'ensemble des portions cultivées obtenues à partir de la culture de toutes les portions issues de la culture complexe initiale.

15. Méthode selon l'une quelconque des revendications précédentes, où la méthode comprend la congélation, le séchage et/ou l'encapsulation des portions cultivées (de l'étape b) ou de la culture complexe assemblée (de l'étape c) ou bien des deux, afin de produire une portion cultivée et/ou une culture complexe assemblée congelée, séchée et/ou encapsulée.

16. Méthode selon l'une quelconque des revendications précédentes, où la méthode comprend l'addition des portions cultivées (de l'étape b) ou bien de la culture complexe assemblée (de l'étape c) à un ingrédient alimentaire afin de fournir un produit alimentaire, tel qu'un produit laitier, un produit alimentaire à base de viande, un produit alimentaire à base de légumes, une composition probiotique ou une composition de bio-conservation.

17. Utilisation de deux, ou plus, portions cultivées, afin de préparer une culture complexe assemblée, chaque portion cultivée étant obtenue après la culture de deux, ou plus, portions - issues d'une culture complexe - dans des conditions de culture différentes qui enrichissent chaque portion en un ou plusieurs microorganismes différents (par exemple des bactéries) et de sorte que chaque portion cultivée confère une fonctionnalité différente.
